# EUROPÄISCHE PATENTSCHRIFT

(11) **EP 0 757 632 B1**
(45) Veröffentlichungstag und Bekanntmachung des Hinweises auf die Patenterteilung: **22.03.2000**
(21) Anmeldenummer: 95905112.9
(22) Anmeldetag: 29.12.1994
(51) Int. Cl.: B60H 3/00, G01N 33/00

(54) **SENSORANORDNUNG ZUR STEUERUNG VON LÜFTUNGSANLAGEN IN FAHRZEUGEN**
SENSOR ARRANGEMENT FOR CONTROLLING VEHICLE VENTILATION SYSTEMS
DISPOSITIF A DETECTEUR POUR LA COMMANDE DE SYSTEMES DE VENTILATION DANS DES VEHICULES

(30) Priorität: 27.04.1994 DE 4414594; 15.10.1994 DE 4436938
(43) Veröffentlichungstag der Anmeldung: 12.02.1997
(73) Patentinhaber: I.T.V.I. INTERNATIONAL TECHNO VENTURE INVEST AG, 9490 Vaduz (LI)
(72) Erfinder: RUMP, Hanns, D-59427 Unna-Massen (DE); PIEPER, Norbert, D-59379 Selm (DE); HILLER, Jörg, D-58300 Wetter (DE); KIESEWETTER, Olaf, D-98716 Geschwenda (DE)
(74) Vertreter: Spalthoff, Adolf, Dipl.-Ing.
(86) Internationale Anmeldenummer: EP9404338
(87) Internationale Veröffentlichungsnummer: WO9529072

(56) Entgegenhaltungen:
- EP-A- 0 327 089
- EP-A- 0 501 127
- DE-A- 3 825 036
- REVUE AUTOMOBILE, Bd. 84,Nr. 4, Januar 1989 BERNE, CH, XP 000009459 'UNE ATMOSPH RE MOINS MALSANE EN VOITURE'

## Beschreibung

Die Erfindung bezieht sich auf eine Sensoranordnung zur Steuerung von Lüftungsanlagen in Fahrzeugen im Um- oder im Zuluftbetrieb in Abhängigkeit von der Schadstoffkonzentration in der Luft außerhalb des Fahrzeugs, mit einem Gassensorelement, dessen elektrischer Widerstand beim Auftreten reduzierender Gase sinkt und beim Auftreten oxidierender Gase steigt, und eine Auswerteeinheit, deren Ausgang an die Steuerung der Lüftungsanlage angeschlossen ist.

Es ist bekannt, daß mittels Gassensorelementen, z.B. Zinndioxydsensorelementen, das Vorhandensein oxidierbarer Gase erfaßt werden kann, z.B. das Vorhandensein von Kohlenmonoxid, Kohlenwasserstoffen und Wasserstoff. Hierbei wird eine gasempfindliche Schicht des Gassensorelements, die beispielsweise aus elektrisch leitendem und beheiztem Zinndioxid bestehen kann, durch das oxidierbare Gas anreduziert; hierdurch ergibt sich eine Verringerung des Ohm'schen Widerstandes der gasempfindlichen Zinndioxidschicht des Gassensorelements. Derartige Gassensorelemente werden in Sensoranordnungen zur Steuerung von Lüftungsanlagen in Fahrzeugen eingesetzt. Immer dann, wenn das Fahrzeug ein Gebiet mit hoher Schadstoffkonzentration erreicht, wird die Lüftungsanlage des Fahrzeugs auf Umluftbetrieb geschaltet, so daß die Schadstoffe von der Fahrgastkabine des Fahrzeugs ferngehalten werden.

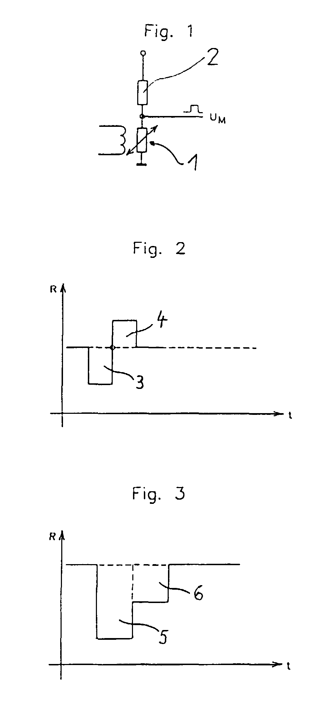

Die bekannten, insbesondere mit Zinndioxidsensorelementen ausgerüsteten Sensoranordnungen reagieren nur sehr gering bei Dieselabgasen, obwohl Dieselabgase die Fahrzeuginsassen subjektiv stärker stören als viele Benzinabgase, z.B. Kohlenmonoxid, weil diese nicht in dem gleichen Ausmaß wie Dieselabgase riechbar sind. Die Ursache für dieses Verhalten liegt in einem sog. "Maskierungseffekt", der deshalb auftritt, weil in Dieselabgasen neben oxidierbaren Gasen in hohem Ausmaß Stickoxide enthalten sind, bei denen es sich um reduzierbare und damit oxidierende Gase handelt. Die in den Dieselabgasen enthaltenen Stickoxide oxidieren die durch oxidierbare Gase der Dieselabgase anreduzierte gasempfindliche Zinndioxidschicht des Gassensorelements wieder auf, so daß sich die reduzierende und die oxidierende Wirkung bei einer Beaufschlagung der gasempfindlichen Zinndioxidschicht in starkem Ausmaß gegenseitig aufheben. Zur Erläuterung dieses "Maskierungseffektes" sei zunächst auf die Figuren 1 bis 3 verwiesen.

Hierbei zeigt Figur 1 eine Sensorschaltung, die ein beheiztes Gassensorelement 1 und einen Außenwiderstand 2 aufweist. Der Außenwiderstand 2 kann auch durch eine Konstantstromquelle ersetzt werden. Die zwischen dem beheizten Gassensorelement 1 und dem Außenwiderstand 2 abgegriffene Teilerspannung UM ist eine Funktion des sich mit der Gasbeaufschlagung ändernden Ohm'schen Widerstandes des beheizten Gassensorelements 1.

Figur 2 zeigt die Veränderung des Ohm'schen Widerstandes des Gassensorelements 1, wenn dieses nur mit reduzierenden oder nur mit oxidierenden Gasen beaufschlagt wird. Der in der Figur dargestellte Impuls 3 ist das Ergebnis einer Beaufschlagung des Gassensorelements mit oxidierbaren Gasen, z.B. mit Kohlenmonoxid (CO). Der Impuls 4 ist das Ergebnis einer Beaufschlagung des Gassensorelements 1 mit reduzierbaren Gasen, z.B. mit Stickoxiden (NOx).

Aus Figur 3 geht der sog. "Maskierungseffekt" hervor; der Impuls 5 wird durch eine Beaufschlagung des Gassensorelements 1 mit oxidierbarem und damit reduzierendem Kohlenmonoxid bewirkt. In Figur 3 folgt auf den Impuls 5 ein Impuls 6, welcher durch ein Mischgas aus Kohlenmonoxid und Stickoxiden bewirkt wird. Trotz des Vorhandenseins der Stickoxide in dem Mischgas bleibt der Ohm'sche Widerstand des Gassensorelements niedriger als in dessen unbeaufschlagtem Zustand. Trotz des Vorhandenseins von reduzierbaren und damit oxidierenden Stickoxiden im Dieselabgas bleibt der Ohm'sche Widerstand des Gassensorelemente 1 unterhalb desjenigen Niveaus, den er im von Abgasen unbelasteten Zustand hat. Dieser Effekt tritt aufgrund den ebenfalls im Dieselabgas enthaltenen oxidierbaren und damit reduzierenden Gasen auf. In Extremfällen kann der Ohm'sche Widerstand des Gassensorelements 1 bei dessen Beaufschlagung mit Mischgas dasselbe Niveau annehmen, was dieser Widerstand auch bei Beaufschlagung des Gassensorelements 1 mit frischer Luft hätte. In jedem Fall ist die Aussagekraft des Gassensorsignals bei einer Beaufschlagung des Gassensorelements 1 mit Mischgasen erheblich reduziert, da sich in dem vorstehend erläuterten Extremfall das Gassensorsignal bei Beaufschlagung des Gassensorelements 1 mit einem Mischgas nicht vom Gassensorsignal bei Beaufschlagung des Gassensorelements mit frischer Luft unterscheidet.

Da somit rein angebotene oxidierbare und reduzierende Gase die Zinndioxidschicht des Gassensorelements niederohmiger und rein angebotene reduzierbare und oxidierende Gase die Zinndioxidschicht des Gassensorelements hochohmiger machen, wurde bisher davon ausgegangen, daß zur sinnvollen Steuerung von Lüftungsanlagen in Fahrzeugen im Um- oder im Zuluftbetrieb zwei Gassensorelemente benötigt werden. Dabei dient ein Gassensorelement zur Erfassung oxidierbarer und damit reduzierender Gase, wohingegen das andere Gassensorelement zur Detektion reduzierbarer und damit oxidierender Gase dient.

Derartige, mit zwei Gassensorelementen ausgerüstete Sensoranordnungen zur Steuerung von Lüftungsanlagen in Fahrzeugen sind vergleichsweise aufwendig. Dies ergibt sich zunächst aus dem Vorhandensein zweier Gassensorelemente. Darüber hinaus müssen die von diesen beiden Gassensorelementen abgegebenen Gassensorsignale weiterverarbeitet und in geeigneter Form zur Auswertung miteinander verknüpft werden, woraus sich eine vergleichsweise komplizierte Ausgestaltung der Weiterverarbeitungs- und Auswerteeinheit ergibt.

Der Erfindung liegt die Aufgabe zugrunde, eine Sensoranordnung zur Steuerung von Lüftungsanlagen in Fahrzeugen im Um- oder im Zuluftbetrieb in Abhängigkeit von der Schadstoffkonzentration in der Luft außerhalb des Fahrzeugs zu schaffen, bei der sowohl die Sensorik als auch die Auswertung einfacher und damit weniger aufwendig gestaltet werden kann.

Diese Aufgabe wird für die eingangs geschilderte Sensoranordnung erfindungsgemäß dadurch gelöst, daß die Sensoranordnung so ausgelegt ist, daß der Anstieg eines in die Auswerteeinheit eingegebenen Gassensorsignals bei einer Erhöhung der Konzentration reduzierender Gase betragsmäßig etwa dem Abfall des in die Auswerteeinheit eingegebenen Gassensorsignals bei einer entsprechenden Erhöhung der Konzentration oxidierender Gase gleichkommt, daß die Auswerteeinheit aus dem in sie eingegebenen Gassensorsignal den Anstieg oder den Abfall desselben je Zeiteinheit ermittelt und daß die Auswerteeinheit ein Schaltsignal zur Verstellung der Lüftungsanlage in den Umluftbetrieb erzeugt, sobald der ermittelte Anstieg oder Abfall des Gassensorsignals je Zeiteinheit betragsmäßig einen festgelegten Wert bzw. Schwellenwert übersteigt.

Bei der erfindungsgemäßen Ausgestaltung der Sensoranordnung wird die Tatsache benutzt, daß bei Lüftungsanlagen in Fahrzeugen im Normalfall mit impulsartigem Auftreten von Verunreinigungen der Luft mit Schadstoffen bzw. Schadgasen gerechnet werden muß und daß statische Verhältnisse in der das Fahrzeug umgebenden Luft im Normalfall nicht vorkommen, da sich zumindest das Fahrzeug in bezug auf die es umgebende Luft bewegt. Des weiteren wird im Falle der erfindungsgemäßen Sensoranordnung ein typisches Verhalten eines Gassensorelements genutzt, das darin besteht, daß das Gassensorelement auf Beaufschlagung mit einem Gas in der Adsorptionsphase typischerweise relativ schnell reagiert, wohingegen die Reaktion in der Desorptionsphase, in deren Verlauf das Gassensorelement seinen ursprünglichen Zustand wieder erreicht, drei- bis fünfmal solange dauert. Das Vorliegen einer Gaskonzentration in der das Fahrzeug umgebenden Luft wird erkannt und die Lüftungsanlage des Fahrzeugs wird in gewünschter Weise auf Umluftbetrieb umgeschaltet, wenn der Anstieg oder der Abstieg des Gassensorsignals je Zeiteinheit einen vorgegebenen Schwellenwert übersteigt. Hierbei ist es unerheblich, ob das Gassensorsignal ansteigt oder absinkt, da die Steilheit des Anstiegs oder des Abfalls betragsmäßig erfaßt und mit dem Schwellenwert verglichen wird. Wird hierbei der Schwellenwert überstiegen, wird das Schaltsignal zur Umschaltung der Lüftungsanlage in den Umluftbetrieb erzeugt.

Um sicherzustellen, daß ein Schaltsignal zur Umschaltung der Lüftungsanlage in den Umluftbetrieb nur dann erzeugt wird, wenn tatsächlich eine spürbare Schadstoffbelastung der das Fahrzeug umgebenden Luft vorliegt, und um ein nicht erforderliches häufiges Umschalten der Lüftungsanlage zu vermeiden, ist es vorteilhaft, wenn die Auswerteeinheit das Schaltsignal nur dann erzeugt, wenn der errechnete Anstieg bzw. Abfall des Gassensorsignals je Zeiteinheit über einen vorgebbaren Zeitraum betragsmäßig den Schwellenwert übersteigt.

Um auch in solchen Fällen, in denen die Schadstoffbelastung der Außenluft langsam - d.h. nicht impulsförmig - ansteigt, zuverlässig zu vermeiden, daß die Luft im Innenraum des Fahrzeugs in derartigen seltenen Situationen eine zu große Qualitätseinbuße erfährt, ist es zweckmäßig, wenn in der Auswerteeinheit aus dem Gassensorsignal für einen bestimmten Zeitraum ein Mittelwert gebildet, diesem Mittelwert durch einen proportionalen Zu- und Abschlag bzw. die Addition und Subtraktion eines Absolutwerts ein definiertes Band zugeordnet und das Schaltsignal erzeugt wird, wenn das Gassensorsignal außerhalb dieses Bandes liegt.

Das erfindungsgemäß angestrebte Schaltverhalten der Lüftungsanlage ist mit vergleichsweise geringem Aufwand erreichbar, wenn die Auswerteeinheit einen Bandpaß aufweist, der ausschließlich ein Amplitudenspektrum passieren läßt, welches einen den Schwellenwert übersteigenden Anstieg bzw. Abfall des Gassensorsignals je Zeiteinheit anzeigt, über den das Gassensorsignal geleitet wird, um einen derartigen Anstieg bzw. Abfall des Gassensorsignals je Zeiteinheit zu ermitteln, und dessen Ausgangssignal zur Erzeugung der Schaltsignale für die Steuerung der Lüftungsanlage herangezogen wird.

Des weiteren ist das erfindungsgemäß angestrebte Schaltverhalten erreichbar, wenn die Auswerteeinheit eine Recheneinrichtung zur Durchführung einer Fourrier-Transformation aufweist, in der das Gassensorsignal rechnerisch auf das Vorhandensein eines bestimmten, einen den Schwellenwert übersteigenden Anstieg bzw. Abfall des Gassensorsignals je Zeiteinheit anzeigenden Amplitudenspektrums untersucht wird, und bei der in der Auswerteeinheit das Schaltsignal für die Steuerung der Lüftungsanlage erzeugt wird, wenn im Gassensorsignal ein derartiges Amplitudenspektrum ermittelt wird.

Eine weitere vorteilhafte Ausführungsform der Erfindung ergibt sich, wenn die Auswerteeinheit ein elektronisches neuronales Netzwerk aufweist, in dem das Gassensorsignal nach Merkmalen auf das Vorhandensein eines den Schwellenwert übersteigenden Anstiegs oder Abfalls des Gassensorsignals je Zeiteinheit untersucht wird, und bei der in der Auswerteeinheit das Schaltsignal erzeugt wird, wenn bei dieser Untersuchung ein den Schwellenwert übersteigender Anstieg oder Abfall des Gassensorsignals je Zeiteinheit ermittelt wird.

Es ist möglich, das elektronische neuronale Netzwerk als drei- oder mehrschichtiges vorwärtsgekoppeltes neuronales Netzwerk auszubilden. Dabei werden sowohl aktuelle wie auch weiter zurückliegende Gassensorsignale bzw. aus diesen Gassensorsignalen errechnete Signale als Eingangsgröße dienen. Der Ausgang dieses neuronalen Netzwerkes liefert dann das zur Steuerung der Lüftungsanlage erforderliche Schaltsignal.

Alternativ zu einem vorwärtsgekoppelten drei- oder mehrschichtigen neuronalen Netzwerk kann auch mit vergleichbaren Ergebnissen ein drei- oder mehrschichtiges rückwärtsgekoppeltes neuronales Netzwerk eingesetzt werden.

Des weiteren ist es möglich, die zur Erreichung des erfindungsgemäß angestrebten Schaltverhaltens erforderlichen Schritte in logischen Regeln zu beschreiben. Die Auswerteeinheit kann dann eine elektronische FUZZY-Logikeinheit aufweisen, mittels der im Gassensorsignal ein den Schwellenwert übersteigender Anstieg oder Abfall des Gassensorsignals je Zeiteinheit erfaßbar ist, wobei in der Auswerteeinheit das Schaltsignal erzeugt wird, wenn bei dieser Untersuchung ein den Schwellenwert übersteigender Anstieg oder Abfall des Gassensorsignals je Zeiteinheit ermittelt wird.

Zweckmäßigerweise ist die Auswerteeinheit so ausgestaltet, daß das Schaltsignal erlischt, wenn das Gassensorsignal innerhalb des dem gebildeten Mittelwert zugeordneten Bandes liegt und im Gassensorsignal kein einen den Schwellenwert übersteigenden Anstieg bzw. Abfall des Gassensorsignals je Zeiteinheit anzeigendes Amplitudenspektrum ermittelt wird. Hierdurch wird sichergestellt, daß die Lüftungsanlage des Fahrzeugs immer dann im Umluftbetrieb arbeitet, wenn der Schadstoffgehalt der das Fahrzeug umgebenden Luft unzulässig hoch ist und wenn ein unzulässig hoher Anstieg des Schadstoffgehalts der das Fahrzeug umgebenden Luft stattfindet.

Es ist außerordentlich wichtig, daß die Rücknahme des Schaltsignals zur Einstellung des Umluftbetriebs bzw. ein Umschaltsignal zum Einschalten des Zuluftbetriebs definiert zu einem Zeitpunkt erzeugt wird, ab dem die Luftqualität außerhalb des Fahrzeugs akzeptabel ist. Diesbezüglich sind verschiedene Verfahrensweisen vorgeschlagen worden, die nicht immer zu günstigen Ergebnissen führen. Als ein Beispiel für den Stand der Technik soll die Verfahrensweise beschrieben werden, bei der die Größe eines durch eine Gasbeaufschlagung des Gassensorelements das Maß für die Betriebszeit der Lüftungsanlage im Umluftbetrieb ist. Hierbei ist ungünstig, daß aufgrund einer kurzzeitigen Schadstoffbeaufschlagung der Luft, die beispielsweise an einer Ampel erzeugt wird, eine vergleichsweise lange Betriebszeit der Lüftungsanlage im Umluftbetrieb folgt, was z.B. dann vollkommen unangemessen ist, wenn das betreffende Fahrzeug den Verursacher dieser Luftverschmutzung, z.B. einen an der Ampel noch vorausfahrenden LKW, nach der Ampel schnell überholt. Andererseits führt die vorstehend beschriebene Verfahrensweise in hochgradig mit Schadstoffen belasteten Tunneln dazu, daß bei Einfahrt in den Tunnel eine dem Anstieg der Schadstoffbefrachtung der Luft entsprechende Betriebszeit im Umluftbetrieb eingestellt wird, wobei die Lüftungsanlage nach Ablauf dieser vorgegebenen Betriebszeit, die von der tatsächlichen Schadstoffbefrachtung der das Fahrzeug umgebenden Luft unabhängig ist, u.U. genau dann auf Zuluftbetrieb umschaltet, wenn sich das Fahrzeug inmitten des Tunnels befindet. Hierdurch wird dann eine besonders hohe Beaufschlagung der Luft im Innenraum des Fahrzeugs mit Schadstoffen bewirkt.

Um eine derartige Verhaltensweise der Steuerung der Lüftungsanlage zu vermeiden, hat die Auswerteeinheit der erfindungsgemäßen Sensoranordnung eine Speichereinrichtung, in der Steigungsumkehrpunkte des Gassensorsignals gespeichert werden, wobei ausgangsseitig der Auswerteeinheit das Schaltsignal erlischt, wenn das Gassensorsignal nicht einem Steigungsumkehrpunkt eine bestimmte vorgebbare Signaldifferenz zum Steigungsumkehrpunkt aufweist und das Gassensorsignal innerhalb des dem gebildeten Mittelwert zugeordneten Bandes liegt.

Von erheblicher Wichtigkeit für eine den Anforderungen der Praxis genügende Anwendung der erfindungsgemäßen Sensoranordnung zur Steuerung von Belüftungsanlagen in Fahrzeugen ist es, die Ansprechempfindlichkeit der Sensoranordnung zuverlässig an die Fahrsituation und die vorgefundenen Bedingungen anzupassen. Hierbei muß die Fähigkeit der menschlichen Nase berücksichtigt werden, die sich ebenfalls an sich ändernde Schadstoffkonzentrationen innerhalb der Luft anpassen kann. Auch die erfindungsgemäße Sensoranordnung soll daher in Bereichen mit überwiegend unbelasteter, sauberer Luft außerordentlich empfindlich auf detektierte Schadstoffe reagieren, wohingegen die Ansprechempfindlichkeit der erfindungsgemäßen Sensoranordnung abgesenkt werden muß, wenn das Fahrzeug überwiegend in Bereichen fährt, in denen die Schadstoffgehalte der Außenluft und damit die Häufigkeit der anzutreffenden Schadstoffspitzen sehr groß ist. Eine solche Änderung der Ansprechempfindlichkeit der erfindungsgemäßen Sensoranordnung ist dringend notwendig, um ein Verhältnis zwischen der Betriebszeit der Lüftungsanlage im Umluftbetrieb und der Betriebszeit der Lüftungsanlage im Zuluftbetrieb zu erreichen, bei dem auch beim Fahren in stark belasteter Außenluft niemals mehr als etwa 50 % der gesamten Zeit die Lüftungsanlage im Umluftbetrieb sich befindet. Nur so kann erreicht werden, daß im Innenraum des Fahrzeugs von den Fahrzeuginsassen abgegebene Feuchtigkeit und von den Fahrzeuginsassen erzeugte Gerüche ud.dgl. zuverlässig aus dem Innenraum des Fahrzeugs ausgetrieben werden. Weiter wird durch eine derartige Betriebsweise verhindert, daß durch zu lange Betriebszeiten der Lüftungsanlage im Umluftbetrieb der Sauerstoffanteil innerhalb des Innenraums des Kraftfahrzeugs auf Werte absinken könnte, die aus medizinischer Sicht bedenklich sind. Bei einem Innenraumvolumen des Fahrgastraums von 2 m3 und bei vollbesetztem Fahrgastraum gilt die Vorgabe, daß die Sauerstoffkonzentration innerhalb der Luft im Fahrgastraum nicht unter 20 % sinken darf; bei normalem Sauerstoffverbrauch der Fahrgäste ergibt sich somit eine maximale Betriebszeit der Lüftungsanlage im Umluftbetrieb von 15 Minuten. Ohne eine automatische Veränderung der Ansprechempfindlichkeit in Abhängigkeit von der aktuellen Verkehrssituation ist das Verhalten einer Sensoranordnung nicht immer zufriedenstellend. Einfluß hat auch die Tatsache, daß die Gassensorelemente von Sensoranordnungen hinsichtlich ihrer spezifischen Empfindlichkeit erhebliche Toleranzen aufweisen, die einen Faktor von bis zu +/- 3 betragen können. Im Verlauf der Lebensdauer der Gassensorelemente kann sich deren Empfindlichkeit darüber hinaus infolge der Begasung mit bestimmten Substanzen ändern. Die Beaufschlagungsverhältnisse der Gassensorelemente sind auch durch Veränderungen von die Gassensorelemente umgebenden Bauteilen möglich.

Außerdem soll berücksichtigt werden, daß sich die Schadstoffbelastung der Luft aufteilt in die Grundbelastung, die beispielsweise in einer Stadt oder in einer Region vorherrscht, und die auftretenden Schadstoffspitzen, die von anderen, vorausfahrenden Fahrzeugen erzeugt werden. Die Grundbelastung wird regelmäßig in ländlichen oder vorstädtischen Bezirken niedriger sein als in dichtbefahrenen Innenstadtregionen.

Zur Kompensation der vorstehend erläuterten Einflußgrößen bzw. zur Kompensation von deren unerwünschter Beeinflussung des Gassensorelements wird gemäß einer vorteilhaften Ausführungsform der Erfindung vorgeschlagen, daß die Auswerteeinheit eine Regeleinrichtung zur Veränderung der Ansprechempfindlichkeit der Sensoranordnung aufweist, mittels der die Ansprechempfindlichkeit der Sensoranordnung bei steigender bzw. sinkender Häufigkeit von den Schwellenwert übersteigenden Anstiegen oder Abfällen des Gassensorsignale je Zeiteinheit reduzier- bzw. erhöhbar ist, wobei der Betrag der Veränderungen der Ansprechempfindlichkeit der Sensoranordnung je Zeiteinheit begrenzt ist.

Eine derartige Variabilität der Ansprechempfindlichkeit der Sensoranordnung kann in konstruktiv wenig aufwendiger Weise verwirklicht werden, wenn die Auswerteeinheit eine Zeitmeßeinrichtung, mittels der die Betriebszeiten der Lüftungsanlage im Zuluft- und im Umluftbetrieb erfaßbar sind, eine Recheneinrichtung, mittels der der Quotient aus der Betriebszeit im Zuluftbetrieb und der Betriebszeit im Umluftbetrieb errechenbar ist, und eine Regeleinrichtung aufweist, mittels der die Ansprechempfindlichkeit der Sensoranordnung bei zunehmendem Umluftbetrieb reduziert und bei zunehmendem Zuluftbetrieb erhöht wird, wobei der Betrag der Veränderung der Ansprechempfindlichkeit der Sensoranordnung je Zeiteinheit begrenzt ist.

In einer weiteren vorteilhaften Ausführungsform mit variabler Ansprechempfindlichkeit weist die Regeleinrichtung zur Veränderung der Ansprechempfindlichkeit der Sensoranordnung einen Außentemperatursensor, der die Lufttemperatur außerhalb des Fahrzeugs erfaßt, einen Innentemperatursensor, der die Lufttemperatur innerhalb des Fahrzeugs erfaßt, und eine Vergleichseinrichtung auf, welche die vom Außentemperatursensor eingegebene äußere Lufttemperatur mit der vom Innentemperatursensor eingegebenen inneren Lufttemperatur vergleicht, wobei die Regeleinrichtung die Ansprechempfindlichkeit der Sensoranordnung erhöht bzw. verringert, wenn die äußere Lufttemperatur größer bzw. kleiner ist als die innere Lufttemperatur.

Die Auswerteeinheit mit der Steuerung der Lüftungsanlage kann als zentraler programmierter Mikroprozessor oder analogtechnisch ausgebildet sein.

Das bei der erfindungsgemäßen Sensoranordnung zum Einsatz kommende Gassensorelement kann als Metalldioxidgassensorelement ausgebildet sein. Hierbei ist zu berücksichtigen, daß die bekannten Zinndioxidgassensorelemente so ausgelegt sind, daß ihre Ansprechempfindlichkeit gegenüber oxidierbaren Gasen besonders ausgeprägt ist, während ihre Ansprechempfindlichkeit gegenüber reduzierbaren Gasen sehr gering ist. Um ein derartiges Zinndioxidgassensorelement im Rahmen der erfindungsgemäßen Sensoranordnung zu verwenden, muß dieses so ausgelegt werden, daß es für oxidierbare und reduzierbare Gase etwa in gleichem Ausmaß empfindlich ist. Dies führt bei den aus dem Stand der Technik bekannten Sensoranordnungen dazu, daß diese Sensoranordnungen besonders anfällig für den vorstehend bereits beschriebenen Maskierungseffekt sind. Im Gegensatz dazu sind für reduzierbare und oxidierbare Gase gleichmäßig empfindliche Zinndioxidgassensorelemente im Falle der erfindungsgemäßen Sensoranordnungen vorteilhaft einsetzbar, da dort die Steilheit des Anstiegs bzw. des Abstiegs des Gassensorsignals - betragsmäßig - erfaßt wird.

Bei der Auslegung der Gassensorelemente für die erfindungsgemäße Sensoranordnung stellte sich heraus, daß grundsätzlich nahezu alle bekannten Metalloxidgassensorelemente, auch Zinndioxidgassensorelemente, bei Beaufschlagung mit oxidierbaren Gasen ihren elektrischen Widerstand verringern und bei Beaufschlagung mit reduzierbaren Gasen ihren elektrischen Widerstand erhöhen. Da das für die erfindungsgemäße Sensoranordnung gewünschte Verhältnis zwischen dem Ansprechverhalten bei einer Beaufschlagung des Gassensorelements mit Dieselabgasen, die im Ausmaß weniger, z.B. 100 bis 200 ppb (Teile je Milliarde) erfolgt und bei einer Beaufschlagung des Gassensorelements mit Benzinabgasen, die im Bereich von wenigen, z.B. 1 bis 50 ppm (Teile je Million) erfolgt, wird bei den bekannten Metalloxidgassensorelementen und Zinndioxidgassensorelementen nicht erreicht.

Für die Verwendung in der erfindungsgemäßen Gassensoranordnung ist das Gassensorelement somit als Mischoxidsensorelement ausgebildet, dessen gasempfindliche Schicht Zinndioxid (SnO2), Wolframtrioxid (WO3), Eisenoxid (Fe2O3), Aluminiumoxid (Al2O3) und als Reaktionsbeschleuniger Platin (Pt) und Palladium (Pd) enthält.

Besonders vorteilhafte Eigenschaften hat das Mischoxidsensorelement, wenn seine gasempfindliche Schicht 29 bis 49, vorzugsweise ca. 39, % Zinndioxid (SnO2), 7 bis 13, vorzugsweise 10, % Eisenoxid (Fe2O3), 28 bis 48, vorzugsweise 38, % Wolframtrioxid (WO3), 7 bis 13, vorzugsweise 10, % Aluminiumoxid (Al2O3), 1 bis 3, vorzugsweise 2, % Palladium (Pd) und 0,5 bis 1,5, vorzugsweise 1, % Platin (Pt) enthält. Es sei jedoch darauf hingewiesen, daß auch andere Mischungsverhältnisse möglich sind, mittels denen ein vergleichbares Ansprechverhalten bei impulsmäßiger Begasung mit Diesel- oder Benzinabgasen erreicht wird.

Das Gassensorelement kann direkt auf einer elektrischen Leiterplatte aufgebracht und mittels Edelmetalldrähten mit der Leiterplatte verbunden sein.

In einer vorteilhaften Ausführungsform ist das Gassensorelement elektrisch mit Platindrähten kontaktiert und in einer Ausnehmung einer elektrischen Leiterplatte der Auswerteeinheit in etwa gleicher Ebene wie diese eingefügt und sind Gassensorelementanschlüsse direkt auf die Leiterplatte aufgeschweißt, so daß das Gassensorelement frei aufgehängt ist.

Metalloxidgassensorelemente verändern ihre Charakteristik, wenn sich die Temperatur an der Sensorelementoberfläche verändert. Bei der Anwendung in der erfindungsgemäßen Sensoranordnung müssen die Metalloxidgassensorelemente und damit deren Sensorelementoberfläche der Außenluft ausgesetzt werden. Hieraus ergibt sich die Problematik, daß bei hohen Fahrgeschwindigkeiten eine direkte Luftbeaufschlagung die Sensorelementoberfläche unzulässig auskühlen würde.

Zur Lösung dieser Problematik wurde vorgeschlagen, das Metalloxidgassensorelement mit einem Labyrinth zu versehen, welches so angeordnet und ausgestaltet ist, daß einerseits die Außenluft das Metalloxidgassensorelement erreicht, andererseits das Metalloxidgassensorelement jedoch ausreichend gegen Feuchtigkeit, Schmutzpartikel und Luftströmungen geschützt ist.

Nachteilig bei einer derartigen Ausgestaltung ist, daß aufgrund der Wegstrecke der Außenluft durch das Labyrinth die Ansprechgeschwindigkeit des Metalloxidgassensorelements verlangsamt wird und daß verschiedene Gase an der vergleichsweise großen Oberfläche des Labyrinths adsorbieren.

Zur Lösung dieser Problematik wurde im Stand der Technik vorgeschlagen, das Metalloxidgassensorelement hinter gasdurchlässigen Sperren anzuordnen, wobei diese Sperren einem Gas einen ungehinderten Zugang zum Metalloxidgassensorelement bieten, wohingegen ein ausreichender Schutz des Sensors gegen Feuchtigkeit, Schmutzpartikel und Luftströmungen gewährleistet ist.

Derartige gasdurchlässige Sperren sind herkömmlicherweise als aufgespannte Membranen ausgebildet, die, sofern sie Luftströmungen ausgesetzt sind, zu Schwingungen angeregt werden. Hierdurch werden Luftbewegungen in dem das Metalloxidgassensorelement umgebenden Bereich erzeugt, die eine Änderung der Temperatur an der Sensorelementoberfläche zur Folge haben. Auf derartige Veränderungen der Temperatur an der Sensorelementoberfläche reagiert das Metalloxidgassensorelement mit einer Änderung seines elektrischen Widerstandes, so daß das Gassensorsignal unzulässig mit einer Störgröße überlagert wird.

Um derartige, die Auswertung erschwerende Beeinflussungen des Gassensorsignals zu verhindern, ist bei der erfindungsgemäßen Sensoranordnung das Gassensorelement in einer Kammer angeordnet, die gegenüber der Auswerteeinheit gasdicht abgeschlossen ist und ein kleines Volumen und eine formstabile und gasdurchlässige Wandung aufweist. Hierdurch kann das erwünschte sehr schnelle Ansprechverhalten des Gassensorelements erreicht werden, da einerseits das Volumen in der das Gassensorelement aufnehmenden Kammer möglichst gering ist und andererseits Luftbewegungen innerhalb der Kammer aufgrund der formstabilen Ausgestaltung der Wandung der Kammer nicht im unerwünschten Ausmaß auftreten können.

Die Wandung der Kammer läßt sich vorteilhaft zumindest teilweise aus einem perforierten Werkstoff herstellen, der zumindest eine Lage eines gasdurchlässigen Materials formstabil trägt und mechanisch stützt.

Es ist auch möglich, die zumindest eine Lage des gasdurchlässigen Materials zwischen zwei Lagen aus Metallgewebe anzuordnen.

Statt zwischen Metallgeweben kann die zumindest eine Lage des gasdurchlässigen Materials auch zwischen zwei formstabilen und perforierten Lagen von Thermoplasten eingebettet sein.

Vorteilhaft ist die zumindest eine Lage des gasdurchlässigen Materials aus Kunststoffolie ausgebildet.

Sofern die Kunststoffolie aus Teflon od.dgl. ausgebildet ist, wird sichergestellt, daß Gas, z.B. Kohlenmonoxid oder Stickoxid, aufgrund der Gasdruckdifferenz durch die Kunststoffolie diffundiert, ohne daß eigentlich ein Lufttransport stattfindet. Alternativ ist auch eine Ausführungsform denkbar, bei der die formstabile und gasdurchlässige Wandung der das Gassensorelement aufnehmenden Kammer aus gesintertem Kunststoff, Glas, Metall od.dgl. ausgebildet ist. Derartige Sinterkörper haben nämlich, je nach Werkstoff, eine Oberfläche, die den Sinterkörper nahezu wasserundurchdringlich macht, wohingegen er Gase passieren läßt.

Für das Ansprechverhalten des Gassensorelements ist es darüber hinaus vorteilhaft, wenn die das Gassensorelement aufnehmende Kammer etwa halbkugelförmig ist.

Die vorstehend erläuterte Anordnung das Gassensorelements in einer Kammer mit den vorstehend geschilderten Eigenschaften hat zur Folge, daß die Reaktionszeit des Gassensorelemente auf eine Änderung der Schadstoffkonzentration in der Außenluft aufgrund der Minimierung des das Gassensorelement umgebenden Raumvolumens ebenfalls minimiert ist, daß das Gassensorelement gegen Luftbewegungen, Staub, Feuchtigkeit, Nässe, Wachse und andere Aerosole, die im KFZ-Betrieb und bei der Wartung vorkommen, gut geschützt ist. Des weiteren ergibt sich durch die Anordnung des Gassensorelements in einer derart ausgestalteten Kammer bei geringen Kosten eine vergleichsweise einfache Bauform.

Bei vielen Anwendungsformen der erfindungsgemäßen Sensoranordnung ist es vorteilhaft, wenn das Gassensorelement und die Auswerteeinheit in einem Gehäuse integriert sind.

Wenn hierbei eine Ausgestaltung gewählt wird, bei der das Gassensorelement und eine Teileinheit der Auswerteeinheit in einem Sensormodul zusammengefaßt sind, die Teileinheit der Auswerteeinheit eine Schwingschaltung, einen Kondensator zur Bestimmung der Frequenz der Schwingschaltung und eine Heizungsregelung zur Regelung der Temperatur des Gassensorelements aufweist und die Schwingschaltung und die Heizungsregelung an ein zentrales Heizungs- und Klimasteuergerät des Fahrzeugs angeschlossen sind, wobei in dem Mikrocomputer des zentralen Heizungs- und Klimasteuergeräts die Weiterverarbeitung eines Ausgangssignals des Sensormoduls voll digital erfolgt, ist es möglich, daß das Gassensorelement einschließlich der ihm unmittelbar zugeordneten Teile der Auswerteeinheit, die einer Minimalauswerteelektronik entsprechen, in einem möglichst kleinen Gehäuse zusammengefaßt werden. Die eigentliche Auswertung der aus dem Sensormodul kommenden Daten findet dann oft in dem ohnehin vorhandenen Mikrocomputer im zentralen Heizungs- und Klimasteuergerät des Kraftfahrzeugs statt. Mit dieser Ausgestaltung des Sensormoduls kann sichergestellt werden, daß das analoge Gassensorsignal zunächst innerhalb des Sensormoduls zu einem digitalen Signal verarbeitet wird und als digitales Signal der eigentlichen Auswertung, die im Mikrocomputer des zentralen Heizungs- und Klimasteuergeräts des Kraftfahrzeugs stattfindet, eingegeben wird. Störanfällige analoge Daten müssen demgemäß nicht transportiert werden und im Mikrocomputer des zentralen Heizungs- und Klimasteuergeräts ist keine aufwendige Analog/Digital-Wandlung erforderlich.

Um das Ansprechverhalten eines aus Metalloxid ausgebildeten Gassensorelements der erfindungsgemäßen Sensoranordnung in bezug auf Dieselabgase und Benzinabgase im vorteilhaften Bereich zu halten, ist es zweckmäßig, wenn das Gassensorelement mittels einer Heizeinrichtung auf einer konstanten Temperatur gehalten wird.

Bei bekannten Sensoranordnungen hat es sich als nachteilig herausgestellt, daß die dort verwendeten Gassensorelemente erhebliche Fertigungstoleranzen aufweisen. Eine elektrisch korrekte Impedanzanpassung ist allerdings immer erst dann gegeben, wenn der elektrische Widerstand des Gassensorelements und sein Außenwiderstand einander entsprechen und die Gassensorspannung der halben Betriebsspannung entspricht. Neben diesen Fertigungstoleranzen haben auch Veränderungen des Widerstands des Gassensorelements, die mit zunehmender Lebensdauer auftreten, außerordentliche Schwierigkeiten bei der zuverlässigen und dauerhaften Erzeugung der Impedanzanpassung zur Folge. Des weiteren werden die bekannten Sensoranordnungen durch die natürliche Feuchte, die von den Werkstoffen aufgenommen wird, unter bestimmten Betriebsumständen und bei Beaufschlagung mit bestimmten Gasen nachteilig beeinflußt. Durch Hydrolyse wird bei Anlegen einer Gleichspannung Wasser dissoziiert. Es kommt zur Ionenbildung und zu einem erheblichen Ionentransport durch das Kristallgitter der gasempfindlichen Schicht des Gassensorelements.

Die vorstehend erläuterten Ursachen können erhebliche Veränderungen der Charakteristik des Gassensorelements zur Folge haben.

Um derartige Folgen zu vermeiden, wird vorgeschlagen, daß der Ohm'sche Widerstand des Gassensorelements Bestandteil einer Schwingschaltung ist und von einer mittelfrequenten Wechselspannung durchflossen wird.

Vorzugsweise wird die Frequenz der Schwingschaltung durch einen Kondensator erzeugt.

In einer besonders vorteilhaften Ausgestaltung der Sensoranordnung weist die Schwingschaltung einen Timerbaustein auf, der mit einem frequenzbestimmenden Kondensator beschaltet ist und einen Rückkopplungszweig aufweist, in dem der Widerstand des Gassensorelements und ein zweiter Widerstand angeordnet sind und der zum Widerstand des Gassensorelements einen Parallelzweig aufweist, in dem ein dritter Widerstand angeordnet ist. Hierdurch kann sichergestellt werden, daß die minimale und die maximale Frequenz auch bei dramatischer Veränderung des Widerstands des Gassensorelements begrenzt wird. Die Verhältnisse des Widerstands des Gassensorelements, des zu diesem in Reihe geschalteten Widerstands und des zu ersterem parallelgeschalteten Widerstands werden so gewählt, daß auch bei extremer Niederohmigkeit oder bei extremer Hochohmigkeit des Gassensorelements bzw. dessen Ohm'schen Widerstands der Gruppenwiderstand der drei Widerstände stets innerhalb des einfach zu berechnenden Bereichs bleibt, was sich unmittelbar auf die Frequenz des Gassensorsignals bezieht. Damit ist sichergestellt, daß sich die Frequenz des Gassensorsignals niemals in einen Bereich verschieben kann, der außerhalb des Aufnahmebereichs der dem Gassensorelement nachgeschalteten Auswerteelektronik liegt.

Im folgenden wird die Erfindung an Hand von Ausführungsformen unter Bezugnahme auf die Zeichnung näher erläutert.

Es zeigen:
- Figur 4: den Verlauf eines Gassensorsignals der erfindungsgemäßen Sensoranordnung;
- Figur 5: den Verlauf des Gassensorsignals bei einer Meßfahrt im Straßenverkehr;
- Figur 6: ein Band, das als Schaltkriterium für die erfindungsgemäße Sensoranordnung dient;
- Figur 7: eine weitere Methode zur Schaffung von Schaltkriterien für die erfindungsgemäße Sensoranordnung;
- Figur 8: eine typische Auswerteeinheit;
- Figur 9: eine weitere Ausführungsform für die Auswerteeinheit der erfindungsgemäßen Sensoranordnung;
- Figur 10: eine Ausführungsform eines in einer Kammer aufgenommenen Gassensorelements;
- Figur 11: eine Ausführungsform der Wandung der Kammer des Gassensorelements gemäß Figur 10;
- Figur 12: eine Draufsicht auf die in Figur 10 dargestellte Hilfsleiterplatte mit Gassensorelement;
- Figur 13: eine Seitenansicht der Hilfsleiterplatte im Schnitt I - I in Figur 12;
- Figur 14: eine Prinzipdarstellung eines Gassensorelements;
- Figur 15: eine Prinzipdarstellung einer ersten Ausführungsform des Gassensorelements der erfindungsgemäßen Sensoranordnung;
- Figur 16: eine Prinzipdarstellung einer weiteren Ausführungsform des Gassensorelements der erfindungsgemäßen Sensoranordnung;
- Figur 17: eine Prinzipdarstellung einer Schaltung zur Trennung einer Grundbelastung von einer dynamisch auftretenden Spitzenbelastung;
- Figur 18 bis Figur 21: Prinzipdarstellungen von Signalverarbeitungsschaltungen zur Regelung der Ansprechempfindlichkeit der erfindungsgemäßen Sensoranordnung;
- Figur 22: eine Darstellung einer Ladekennlinie eines RC-Gliedes; und
- Figur 23: eine Prinzipdarstellung eines Sensormoduls der erfindungsgemäßen Sensoranordnung.

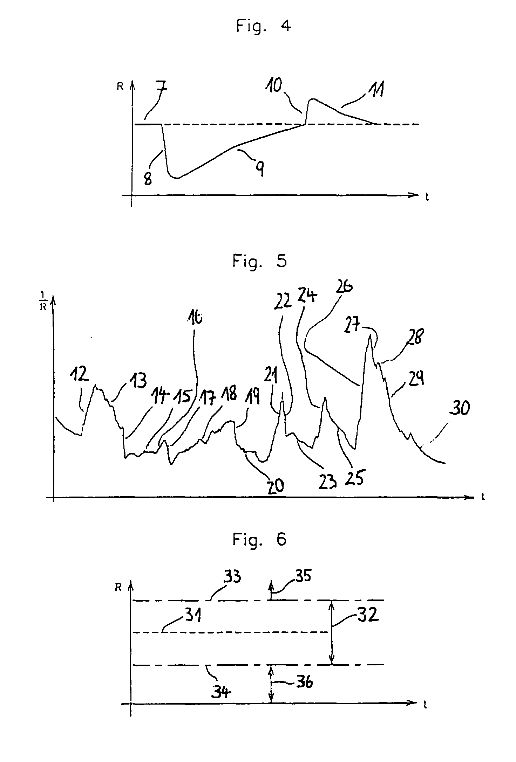

Aus dem in Figur 4 dargestellten Verlauf des Gassensorsignals des erfindungsgemäßen Gassensorelements gehen diejenigen Zusammenhänge hervor, welche bei der Ausgestaltung der erfindungsgemäßen Sensoranordnung Berücksichtigung finden. Das Gassensorsignal ist in Figur 4 als Widerstandswert eines Ohm'schen Widerstandes R über der Zeit t dargestellt. Zunächst nimmt das Gassensorsignal den Wert 7 an, der sich ergibt, wenn die Außenluft weder von oxidierenden noch von reduzierenden Gasen belastet ist. Dieser Wert 7 wird bei Auftreten oxidierbarer Gase mit einer großen negativen Stelgung verlassen, wie sich aus dem Verlauf des Gassensorsignals in der Phase 8 ergibt. Hierbei wird der Widerstand des Gassensorelements niederohmiger, wobei das der Steigung entsprechende Verhältnis zwischen der Widerstandsänderung und der ablaufenden Zeit besteht. Auf die Beaufschlagung des Gassensorelements mit oxidierbaren Gasen, welche insbesondere in Benzinabgasen enthalten sind, folgt eine sog. Desorptionsphase 9, in der das Gassensorelement durch unbelastete Außenluft bewegt wird und während der das Gassensorelement desorbiert. Diese Desorptionsphase dauert bis zu dem Zeitpunkt, zu dem das Gassensorsignal wieder seinen Wert 7 für unbelastete Luft erreicht hat. Während der Desorptionsphase 9 ist die positive Steigung des Gassensorsignals erheblich geringer als die negative Steigung des Gassensorsignals während der auch Adsorptionsphase genannten Phase 8. Das Verhältnis der Widerstandsänderung je Zeiteinheit ist während der Desorptionsphase 9 deutlich kleiner als während der Adsorptionsphase 8.

Nachdem das Gassensorsignal wieder seinen unbelastete Außenluft entsprechenden Wert 7 eingenommen hat, durchläuft das Gassensorelement einen Bereich, in dem die Außenluft mit reduzierbaren Gasen befrachtet ist. Aufgrund der Beaufschlagung des Gassensorelements mit diesen reduzierbaren Gasen folgt eine Adsorptionsphase 10, in deren Verlauf der Widerstandswert des Gassensorelements aufgrund der oxidierenden Wirkung der reduzierbaren Gase stark erhöht wird. Die positive Steigung des Gassensorsignals während der Adsorptionsphase 10 entspricht etwa der negativen Steigung des Gassensorelements während der Adsorptionsphase 8. Nachdem der Bereich der mit reduzierbaren Gasen befrachteten Außenluft durchfahren ist, beginnt eine Desorptionsphase 11, in der die gasempfindliche Schicht des Gassensorelements desorbiert. Die Desorptionsphase läuft in der jetzt unbelasteten Außenluft, bis das Gassensorsignal wieder den ursprünglichen Wert 7 eingenommen hat. Die negative Steigung, die sich aus der Veränderung des Widerstandswerts je Zeiteinheit ergibt, gleicht während der Desorptionsphase 11 betragsmäßig der positiven Steigung während der vorangegangenen Desorptionsphase 9. Aus Figur 4 geht hervor, daß die Veränderung des Widerstandswerts je Zeiteinheit während der Desorptionsphasen 9, 11 deutlich niedriger liegt als während der Adsorptionsphasen 8, 10.

Die Steigung des Gassensorsignals ist während der Adsorptionsphase eines mit oxidierbaren Gasen beaufschlagten Gassensorelements und während der Desorptionsphase 11 eines mit reduzierbaren Gasen beaufschlagten Gassensorelements negativ; positiv ist diese Steigung während der Desorptionsphase 9 eines mit oxidierbaren Gasen beaufschlagten Gassensorelements und während der Adsorptionsphase 10 eines mit reduzierbaren Gasen beaufschlagten Gassensorelements.

Kennzeichnend für den Verlauf des Gassensorsignals sind die betragsmäßig stark unterschiedlichen Steigungswerte während der Adsorptionsphasen 8, 10 und der Desorptionsphasen 9, 11. Diese Eigenschaft des Gassensorsignals wird im Falle der vorliegenden Erfindung benutzt, um zweckdienliche Schaltkriterien aufzustellen.

Sofern ein Abfall des Gassensorsignals mit einer einen vorgegebenen Schwellenwert übersteigenden Änderung des Gassensorsignals je Zeiteinheit festgestellt wird, ist das Gassensorelement mit einem oxidierbaren Gas beaufschlagt worden. Sofern ein Anstieg des Gassensorsignals mit einer den Schwellenwert überschreitenden Änderung des Gassensorsignals je Zeiteinheit festgestellt wird, ist das Gassensorelement mit einem reduzierbaren Gas beaufschlagt worden. Die vorstehenden Regeln gelten für jede Änderung des Gassensorsignals, unabhängig davon, in welche Richtung sich das Gassensorsignal verändert, und auch unabhängig davon, welches Ausgangsniveau das Gassensorsignal zum Zeitpunkt des Auftretens eines entsprechenden Anstiegs oder eines entsprechenden Abfalls aufweist.

Bei einer Fahrt im Straßenverkehr treten meist nicht solche Verhältnisse auf, bei denen das Gassensorelement nach jeder Beaufschlagung mit reduzierbaren oder oxidierbaren Gasen und der sich daraus ergebenden Adsorptionsphase zunächst in einer Desorptionsphase wieder zurück in seinen ursprünglichen Zustand gelangen kann, den es bei Beaufschlagung mit schadstofffreier Luft einnimmt. Vielmehr erfolgen im Straßenverkehr Beaufschlagungen mit reduzierbaren oder oxidierbaren Gasen während auf andere Beaufschlagungen beruhenden Adsorptions- und Desorptionsphasen. Ein typischer Verlauf des Gassensorsignals im Straßenverkehr ist in Figur 5 dargestellt. Dort ist der Kehrwert des Widerstandswerts des Gassensorelements über die Zeit aufgetragen.

Zu Beginn wird das Gassensorelement mit aus Benzinabgasen stammenden oxidierbaren Gasen beaufschlagt, woraus sich die eine große Steigung aufweisende Adsorptionsphase 12 ergibt. Auf das Ende der Adsorptionsphase 12, das vor Erreichen der Sättigung der gasempfindlichen Schicht des Gassensorelements mit oxidierbaren Gasen erfolgt, folgt eine Desorptionsphase 13. Während dieser Desorptionsphase 13 desorbiert die gasempfindliche Schicht des Gassensorelements, wobei die Desorptionsgeschwindigkeit deutlich geringer ist als die Adsorptionsgeschwindigkeit während der Adsorptionsphase 12. Hieraus ergibt sich, daß die negative Steigung des Gassensorsignals während der Desorptionsphase 13 betragsmäßig deutlich geringer ist als die positive Steigung des Gassensorsignals während der Adsorptionsphase 12. Während der Desorptionsphase 13 wird das Gassensorelement mit reduzierbaren Gasen, z.B. NO und NOx, beaufschlagt, die in Dieselabgasen enthalten sind. Hierauf folgt eine Adsorptionsphase 14 mit einer großen Steigung, wobei die Steigung der Adsorptionsphase 14 betragsmäßig der Steigung der vorangegangenen Adsorptionsphase 12 entspricht. Hinsichtlich des Gassensorsignalverlaufs unterscheidet sich die Adsorptionsphase 14 insoweit von der Desorptionsphase 13, als die Steigung der Adsorptionsphase 14 betragsmäßig deutlich größer ist als die Steigung während der Desorptionsphase 13.

Nach Beendigung der Adsorptionsphase 14, die vor Sättigung der gasempfindlichen Schicht des Gassensorelements mit reduzierbaren Gasen stattfindet, folgt eine Desorptionsphase 15, eine geringfügige Beaufschlagung des Gassensorelements mit oxidierbaren Gasen und die daraus folgende kleine Adsorptionsphase 16, unmittelbar darauf folgend eine kurzfristige Beaufschlagung des Gassensorelemente in erheblicher Höhe mit reduzierbaren Gasen, die aus den Dieselabgasen eines Lastkraftwagens stammen können, und die sich daraus ergebende Adsorptionsphase 17, eine Desorptionsphase 18, eine erneute Beaufschlagung mit reduzierbaren Gasen, die zur Adsorptionsphase 19 führt, eine Desorptionsphase 20, eine Beaufschlagung mit oxidierbaren Gasen, die zur Adsorptionsphase 21 führt, eine Beaufschlagung mit reduzierbaren Gasen, die zur Adsorptionsphase 22 führt, eine Desorptionsphase 23, eine Beaufschlagung des Gassensorelements mit oxidierbaren Gasen, die zur Adsorptionsphase 24 führt, eine Desorptionsphase 25, eine erneute Beaufschlagung des Gassensorelements mit oxidierbaren Gasen, die zur Adsorptionsphase 26 führt, eine Beaufschlagung des Gassensorelements mit reduzierbaren Gasen, die zur Adsorptionsphase 27 führt, eine kurze Desorptionsphase 28, eine erneute Beaufschlagung des Gassensorelements mit reduzierenden Gasen, die zur Adsorptionsphase 29 führt, und eine Desorptionsphase 30.

Aus der Darstellung des Gassensorsignals gemäß Figur 5 geht eindeutig hervor, daß die Steigung in den Adsorptionsphasen betragsmäßig jeweils deutlich größer ist als die Steigung in den Desorptionsphasen. Das Prinzip der vorliegenden Erfindung beruht nunmehr darauf, für die Steigung des Gassensorsignals einen Schwellenwert vorzugeben, mit der Folge, daß eine Umschaltung der Lüftungsanlage des Fahrzeugs vom Zuluft- auf den Umluftbetrieb immer dann erfolgt, wenn die Steigung des Gassensorsignals betragsmäßig diesen Schwellenwert übersteigt.

Mit dem vorstehend erläuterten Schaltverhalten der Lüftungsanlage läßt sich vermeiden, daß im Straßenverkehr häufig vorkommende plötzliche Verschlechterungen der Qualität der das Fahrzeug umgebenden Luft in den Innenraum des Fahrzeugs gelangen. Dieses Schaltverhalten ist jedoch nicht geeignet, um bei einem allmählichen Anstieg der Schadstoffbelastung der das Fahrzeug umgebenden Luft, der beispielsweise in Tunneln, in statischen Verkehrssituationen od.dgl. auftreten kann, zu verhindern, daß diese Verschlechterung der Qualität der das Fahrzeug umgebenden Luft auch Auswirkungen auf die Qualität der Luft im Innenraum des Fahrzeugs hat.

Um auch bei so verlaufenden Qualitätsverschlechterungen der das Fahrzeug umgebenden Luft zu verhindern, daß sich die Luft innerhalb des Fahrzeugs unzulässig verschlechtert, wird aus dem Gassensorsignal durch Integration od.dgl. für einen bestimmten Zeitraum rechnerisch ein Mittelwert 31 erzeugt, der in Figur 6 dargestellt ist. Um diesen Mittelwert 31 herum wird ein Band 32 erzeugt, was durch Subtraktion und Addition eines Absolutwerts oder durch einen auf den Mittelwert 31 bezogenen Proportionalzu- und -abschlag geschehen kann. Dieses dem Mittelwert 31 zugeordnete Band 32 wird durch den oberen Wert 33 und den unteren Wert 34 begrenzt, wobei der obere Wert 33 und der untere Wert 34 kennzeichnen die positive bzw. negative proportionale oder absolute noch erlaubte Abweichung des Gassensorsignals vom Mittelwert 31.

Ein Schaltsignal zum Umschalten der Lüftungsanlage des Fahrzeugs von Zuluft- auf Umluftbetrieb erfolgt immer dann, wenn das aktuelle Gassensorsignal außerhalb des Bandes 32 und damit innerhalb der Bereiche 35 oder 36 liegt.

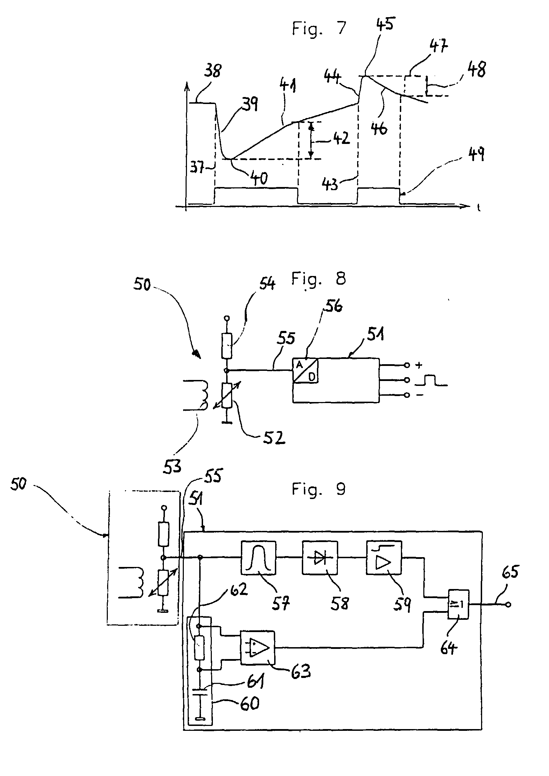

Das Signal zur Umschaltung der Lüftungsanlage von Zuluft- auf Umluftbetrieb muß zu einem Zeitpunkt erzeugt werden, von dem an die Qualität der das Fahrzeug umgebenden Luft akzeptabel ist. Um ein derartiges Schaltverhalten zu erreichen, wird auf Umluftbetrieb umgeschaltet, wenn - wie sich aus Figur 7 ergibt - zu einem Zeitpunkt 37 ein Abstieg des Gassensorsignals beginnt, wobei die negative Steigung dieses Abstiegs des Gassensorsignals größer ist als der vorstehend bereits erwähnte Schwellenwert. Vor dem Zeitpunkt 37 verläuft das Gassensorsignal z.B. auf seinem Normalwert 38, den es bei unbelasteter Außenluft einnimmt. Während der auf den Zeitpunkt 37 folgenden Adsorptionsphase 39 sinkt das Gassensorsignal bis zu einem Umkehrpunkt 40, mit dem die auf die Adsorptionsphase 39 folgende Desorptionsphase 41 beginnt. Im Umkehrpunkt 40 wechselt das Vorzeichen der Steigung des Gassensorsignals von einem negativen Vorzeichen während der Adsorptionsphase 39 zu einem positiven Vorzeichen während der Desorptionsphase 41. Durch diesen Vorzeichenwechsel der Steigung des Gassensorsignals wird der Umkehrpunkt 40 erkannt und in einer Auswerteeinheit gespeichert. Des weiteren wird diesem Umkehrpunkt 40 das Gassensorsignal zum Zeitpunkt der Umkehr des Vorzeichens der Steigung des Gassensorsignale zugeordnet. In der Auswerteeinheit ist ein frei vorgebbarer Differenzwert 42 abgespeichert. Wenn das Gassensorsignalniveau während der Desorptionsphase 41 ausgehend vom Umkehrpunkt 40 um den Differenzwert 42 angestiegen ist, erfolgt eine Umschaltung der Lüftungsanlage des Fahrzeugs vom Umluft- auf den Zuluftbetrieb. Vor Einleitung dieser Umschaltung von Umluft- auf Zuluftbetrieb wird von der Auswerteeinheit geprüft, ob sich das Gassensorsignal innerhalb des sich aus Figur 6 ergebenden Bandes 32 befindet.

Im Zeitpunkt 37 erfolgte eine Beaufschlagung der gasempfindlichen Schicht des Gassensorelements mit einem oxidierbaren Gas. Nach Beendigung der Desorptionsphase 41 erfolgt nunmehr eine Beaufschlagung der gasempfindlichen Schicht des Gassensorelements mit einem reduzierbaren Gas. Zu Beginn dieser Beaufschlagung im Zeitpunkt 43 wechselt die Steigung des Gassensorsignals von dem vergleichsweise geringen Wert während der Desorptionsphase 41 auf den vergleichsweise großen Wert während der auf den Zeitpunkt 43 folgenden Adsorptionsphase 44. Die Steigung des Gassensorsignals während der Adsorptionsphase 44 übertrifft den vorgegebenen Schwellenwert, so daß zum Zeitpunkt 43 eine Umschaltung der Lüftungsanlage des Fahrzeugs vom Zuluft- auf den Umluftbetrieb erfolgt. Die Adsorptionsphase 44 geht nach Beendigung der Beaufschlagung der gasempfindlichen Schicht des Gassensorelements mit reduzierbaren Gasen in einem Umkehrpunkt 45 in eine Desorptionsphase 46 über. Im Umkehrpunkt 45 zwischen der Adsorptionsphase 44 und der Desorptionsphase 46 ändert sich das Vorzeichen der Steigung des Gassensorsignals. Durch diese Vorzeichenänderung wird der Umkehrpunkt 45 in der Auswerteeinheit erkannt und dann gespeichert. Darüber hinaus wird das Gassensorsignalniveau 47 im Umkehrpunkt 45 in der Auswerteeinheit gespeichert. Des weiteren ist in der Auswerteeinheit ein Differenzwert 48 gespeichert, der frei vorgebbar ist. Der Differenzwert 48 für Desorptionsphasen 46 nach einer Beaufschlagung mit reduzierbaren Gasen kann dem Differenzwert 42 für eine Desorptionsphase 41 nach einer Beaufschlagung mit oxidierbaren Gasen entsprechen, er kann sich jedoch auch von diesem unterscheiden, da Dieselabgase anders empfunden werden als Benzinabgase.

Wenn das Gassensorsignal, ausgehend vom Umkehrpunkt 45, während der Desorptionsphase 46 um den Differenzwert 48 unter das Gassensorsignalniveau 47 im Umkehrpunkt 45 absinkt, erfolgt eine Umschaltung der Lüftungsanlage des Fahrzeugs vom Umluft- in den Zuluftbetrieb.

Der Graph 49 zeigt den Betriebszustand der Lüftungsanlage an. Aus dem Graph 49 ergibt sich, daß die Lüftungsanlage vom Zeitpunkt 37 bis zur Umschaltung in den Zuluftbetrieb nach Anstieg um den Differenzwert 42 in der aus den Umkehrpunkt 40 folgenden Desorptionsphase 41 und zwischen dem Zeitpunkt 43 und dem Absinken um den Differenzwert 48 in der auf den Umkehrpunkt 45 folgenden Desorptionsphase 46 im Umluftbetrieb gearbeitet hat.

In Figur 8 ist der grundsätzliche Aufbau der erfindungsgemäßen Sensoranordnung dargestellt.

Zu der Sensoranordnung gehören ein Sensorteil 50 und eine Auswerteeinheit 51.

Das Sensorteil 50 hat ein Gassensorelement 52, das einen veränderbaren Ohm'schen Widerstand aufweist und durch eine Heizeinrichtung 53 auf einer Betriebstemperatur unterhalb 250 Grad C gehalten wird. Des weiteren gehört zum Sensorteil 50 ein Außenwiderstand 54. Ein zwischen dem Gassensorelement 52 und dem Außenwiderstand 54 abgegriffenes Gassensorsignal wird durch eine Verbindungsleitung 55 in die Auswerteeinheit 51 eingegeben. Diese zentrale Auswerteeinheit 51 dient sowohl zur Auswertung des ihr durch die Verbindungsleitung 55 eingegebenen Gassensorsignals als auch zur Steuerung der in Figur 8 nicht dargestellten Lüftungsanlage des Fahrzeugs. Die Auswerteeinheit 51 weist einen integrierten Analog/Digital-Wandler 56 auf, der das analog in die Auswerteeinheit 51 eingegebene Gassensorsignal in ein digitales Signal umwandelt. Des weiteren ist in der Auswerteeinheit 51 ein Programmablauf digital gespeichert, mittels dem gemäß den an Hand der Figuren 4 bis 7 erläuterten Schaltkriterien ein Schaltsignal für die Umschaltung der Lüftungsanlage aus dem Zuluft- in den Umluftbetrieb und umgekehrt erzeugt wird.

Bei der in Figur 9 dargestellten Ausführungsform der Sensoranordnung entspricht das Sensorteil 50 dem an Hand der Figur 8 vorstehend beschriebenen Sensorteil 50. Aus dem Sensorteil 50 gelangt das Gassensorsignal durch die Verbindungsleitung 55 in die Auswerteeinheit 51.

In der Auswerteeinheit 51 wird das Gassensorsignal über einen Bandpaß 57 geführt. Der Bandpaß 57 ist so ausgelegt, daß nur solche Frequenzbänder ihn passieren können, die für einen Verlauf des Gassensorsignals typisch sind, wenn das Gassensorsignal sich entsprechend einer am Gassensorelement 52 stattfindenden Adsorptionsphase verändert. Das heißt, den Bandpaß 57 können nur solche Frequenzbänder passieren, die eine den vorgegebenen Schwellenwert betragsmäßig übertreffende Steigung des Gassensorsignals kennzeichnen. Diesbezüglich sei noch darauf hingewiesen, daß das Gassensorelement 52 in einer später noch zu beschreibenden Weise so ausgelegt ist, daß die Steigung bei seiner Beaufschlagung mit oxidierbaren Gasen und der sich daraus ergebenden Adsorptionsphase gleich der Steigung bei seiner Beaufschlagung mit reduzierbaren Gasen und der sich daraus ergebenden Adsorptionsphase ist.

In einem Demodulator 58 wird das Ausgangssignal des Bandpasses 57 gleichgerichtet. Das gleichgerichtete Ausgangssignal des Demodulators 58 triggert bei entsprechender Größe einen Komparator 59 mit einer frei wählbaren Schaltwelle. Hierdurch wird immer dann ein Schaltsignal für die Lüftungsanlage gewonnen, wenn das mit der erfindungsgemäßen Sensoranordnung ausgerüstete Fahrzeug Bereiche mit schadstoffbelasteter Außenluft durchfährt, wobei das Schaltverhalten der sensoranordnung unabhängig davon ist, ob die Schadstoffbelastung der Außenluft auf Diesel- oder Benzinabgase zurückgeht.

Dieses Schaltsignal steht am Ausgang des Komparators 59 solange an, solange sich das Gassensorelement 52 des Sensorteils 50 in der Adsorptionsphase befindet. Eine Sättigung des Gassensorelements 52 kommt in der Praxis nahezu niemals vor, da die weitaus überwiegende Mehrzahl der auftretenden Schadstoffbelastungen der Außenluft relativ kurzzeitiger Natur ist, was auch darauf zurückgeht, daß sich im Normalbetrieb das Kraftfahrzeug in bezug auf die Außenluft bewegt. Adsorptionsphasen dauern allenfalls einige wenige Minuten, so daß das Gassensorelement 32 normalerweise nicht in einen gesättigten Zustand geführt wird.

Es gibt jedoch Verkehrssituationen, in denen eine Sättigung des Gassensorelements 52 nicht immer vermieden werden kann. Eine Führung des Gassensorelements 52 in den Bereich der Sättigung erfolgt beispielsweise dann, wenn sich das Fahrzeug über einen längeren Zeitraum in einer Umgebung mit sehr stark schadstoffbelasteter Außenluft befindet. In einem solchen Fall wird die Steigung des Gassensorsignals gegen Null gehen, obwohl die Schadstoffbelastung der Außenluft gleichbleibt oder sich sogar noch erhöht. Ohne weitere Vorkehrungen würde die Lüftungsanlage des Fahrzeugs in einer derartigen Verkehrssituation von Umluft- auf Zuluftbetrieb umschalten, da das Gassensorsignal in einer derartigen Verkehrssituation diejenigen den Bandpaß 57 passierenden Frequenzbänder nicht mehr aufwiese.

Da beispielsweise in Verkehrstunneln und dichten Staus solche Situationen auftreten können, wird in der Auswerteeinheit 51 auch das tatsächliche Niveau des Gassensorsignals erfaßt und verarbeitet. Dazu wird das durch die Verbindungsleitung 55 in die Auswerteeinheit 51 eingegebene Gassensorsignal einem RC-Glied eingegeben, mittels dem es mit einer hohen Zeitkonstante integriert wird. An einem Kondensator 61 des RC-Glieds 60 bildet sich demgemäß ein Mittelwert des Gassensorsignals aus. Immer dann, wenn das Gassensorsignal einen höheren Wert einnimmt als der am Kondensator 61 des RC-Glieds 60 vorhandeze Mittelwert, bildet sich über einen Ladewiderstand 62 des RC-Glieds eine Spannung, die der Differenz zwischen dem aktuellen Gassensorsignal und dem am Kondensator 61 des RC-Glieds vorhandenen Mittelwert des Gassensorsignals entspricht. Diese Spannung wird einem Komparator 63 zugeführt, hierdurch wird immer dann ein Schaltsignal erzeugt, wenn das aktuelle Gassensorsignal größer ist als der im Kondensator 61 des RC-Glieds 60 quasi "gespeicherte" Mittelwert.

Bei entsprechender Auswahl der Zeitkonstante des RC-Glieds 60 wird in den vorstehend erläuterten Verkehrssituationen, bei denen die das Fahrzeug umgebende Luft dauerhaft hohe Schadstoffkonzentrationen aufweist und daher das Gassensorelement 52 gesättigt sein kann, dennoch gewährleistet, daß die Lüftungsanlage des Fahrzeugs weiterhin im Umluftbetrieb bleibt.

Auch bei dauerhaften sehr hohen Schadstoffbelastungen der das Fahrzeug umgebenden Luft ist es jedoch erwünscht, daß nach Ablauf eines bestimmten Zeitraums die Lüftungsanlage wieder vom Umluft- auf den Zuluftbetrieb umschaltet; anderenfalls würde, bedingt durch den Sauerstoffverbrauch der Insassen des Fahrzeugs und durch die erzeugte Feuchtigkeit sowie die von ihnen erzeugten und abgegebenen Stoffwechselprodukte - die Qualität der Luft im Innenraum des Fahrzeugs rapide nachlassen. Um dies zu verhindern, wird in der Auswerteeinheit der Zeitpunkt, an dem die Lüftungsanlage vom Zuluft- auf den Umluftbetrieb umschaltet, erfaßt. Nach Ablauf eines vorgegebenen Zeitraums, dessen Länge sich auf dem Volumen des Innenraums des Fahrzeugs sowie aus der ebenfalls erfaßten Anzahl der Fahrzeuginsassen ergeben kann, wird die Lüftungsanlage zwangsweise vom Umluft- auf den Zuluftbetrieb umgestellt, falls während dieses Zeitraums nicht aufgrund der Änderung der Schadstoffbelastung der das Fahrzeug umgebenden Außenluft eine Umschaltung der Lüftungsanlage vom Umluft- auf den Zuluftbetrieb erfolgt ist. Das Ausgangssignal des Komparators 59 und das Ausgangssignal des Komparators 63 der Auswerteeinheit 51 werden in einem Verknüpfungsglied 64 miteinander verknüpft. Das Ausgangssignal des Verknüpfungsglieds 64 wird durch eine Ausgangsleitung 65 des Verknüpfungsglieds 64 bzw. der Auswerteeinheit 51 als Schaltsignal in die Steuerung der Lüftungsanlage eingegeben.

Bei einer bevorzugten Ausführungsform der erfindungsgemäßen Sensoranordnung werden die Schaltungsfunktionen, die vorstehend mittels analoger Funktionsgruppen erläutert wurden, rechnerisch nachgebildet. Hierbei ergibt sich dann ein mikroprozessorgesteuerter Ablauf, um, entsprechend dem vorstehend erläuterten erwünschten Schaltverhalten, Schaltsignale für die Umschaltung der Lüftungsanlage des Fahrzeugs aus dem Zuluft- in den Umluftbetrieb oder umgekehrt zu erhalten.

Das Gassensorelement 52 ist vorteilhaft als Mischoxidsensorelement ausgebildet, wobei seine gasempfindliche Schicht zu ca. 39 % Zinndioxid, ca. 10 % Eisenoxid, ca. 38 % Wolframtrioxid, ca. 10 % Aluminiumoxid, ca. 2 % Palladium und ca. 1 % Platin enthält, wobei das Palladium und das Platin als Reaktionsbeschleuniger fungieren.

Bei einer bevorzugten Ausführungsform der erfinderischen Sensoranordnung wird das Gassensorelement bei einer Temperatur betrieben, die unterhalb 200 Grad C liegt. Bei einer derartigen Betriebstemperatur des Gassensorelements 52 ist dessen Ansprechempfindlichkeit gegenüber oxidierbaren Gasen erheblich verringert. Die Ansprechempfindlichkeit des Gassensorelements 52 gegenüber reduzierbaren Gasen ist jedoch vergleichbar mit der Ansprechempfindlichkeit bei anderen Temperaturen. Hierdurch wird erreicht, daß bei hinsichtlich ihres Störpotentials für die Fahrzeuginsassen vergleichbaren Beaufschlagungen des Gassensorelements 52 mit reduzierbaren und oxidierbaren Gasen etwa gleiche, wenn auch in unterschiedliche Richtungen verlaufende Veränderungen des Gassensorsignals auftreten. Aufgrund dieses gleichmäßigen Ansprechverhaltens des Gassensorelements 52 gegenüber oxidierbaren und reduzierbaren Gasen ist es möglich, die Änderung des Gassensorsignals je Zeiteinheit, d.h. den Betrag der Steigung des Gassensorsignals als Schaltparameter für die Lüftungsanlage zu nutzen.

Bei einer weiteren bevorzugten Ausführungsform der erfindungsgemäßen Sensoranordnung sind das Sensorteil 50 sowie die Auswerteeinheit 51 in einem innen gemeinsamen Gehäuse angeordnet.

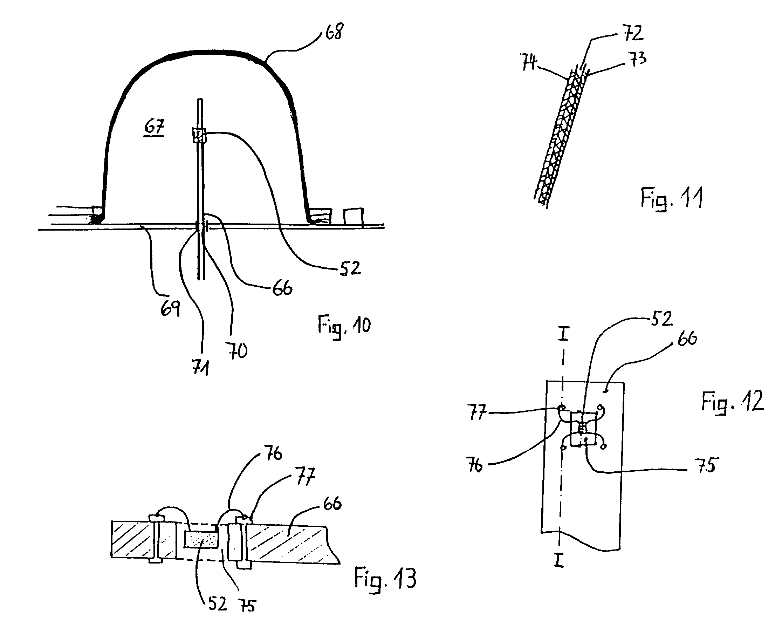

In einem in Figur 10 dargestellten Ausführungsbeispiel ist das Gassensorelement an einer Leiterplatte 66 angebracht und innerhalb einer Kammer 67 angeordnet. Die Kammer 67 wird durch eine Wandung bzw. Abschirmung 68 und durch eine Horizontalplatte 69 gebildet. Die Horizontalplatte 69 hat eine in ihrem Querschnitt etwa demjenigen der Leiterplatte 66 entsprechende Öffnung 70, durch die hindurch die Leiterplatte 66 in die Kammer 67 vorsteht. Zur Abdichtung ist zwischen dem Innenumfang der Öffnung 70 und dem sich innerhalb der Öffnung befindenden Abschnitt der Leiterplatte 66 Abdichtungsmaterial 71 vorgesehen. Das Gassensorelement 52 ist hierbei unmittelbar an der Leiterplatte 66 angebracht, wobei die Leiterplatte 66 die Verbindung zur in Figur 10 nicht dargestellten Auswerteeinheit 51 herstellt. Bei diesem Ausführungsbeispiel ist die Auswerteeinheit 51 in einem separaten Gehäuse untergebracht. Durch die Horizontalplatte 69 und das Abdichtungsmaterial 71 sind die Kammer 67 und der die Auswerteeinheit 51 aufnehmende, in Figur 10 nicht dargestellte Innenraum des betreffenden Gehäuses hermetisch voneinander abgetrennt. Eine derartige hermetische Abtrennung ist erforderlich, um beispielsweise die Abklingzeiten des Gassensorelements 52 zu minimieren und um Störfaktoren auszuschließen, die sich beispielsweise aus dem Ausgasen sich erwärmender elektronischer Bauteile der Auswerteeinheit 51 oder sich erwärmender Kunststoffteile ergeben könnten.

Die Wandung bzw. Abschirmung 68, die die das Gassensorelement 52 aufnehmende Kammer 67 umgibt, ist gasdurchlässig ausgebildet. Somit ist ein ungehinderter und schneller Gasaustausch zwischen der Kammer 67 und der die Wandung bzw. Abschirmung 68 umgebenden Luft möglich ist.

Die Wandung 68 hat als einen Bestandteil eine gasdurchlässige Folie 72. Als besonders geeigneter Werkstoff für die Folie hat sich Teflon herausgestellt.

Die Folie 72 ist, wie sich insbesondere aus Figur 11 ergibt, beidseitig durch formstabile und perforierte Wandungsteile 73, 74 abgedeckt und gestützt. Sie ist zwischen den Wandungsteilen 73, 74 so eingespannt, daß eine Vibration der Folie 72 in jedem Fall verhindert wird.

Die Wandungsteile 73, 74 können beispielsweise als tiefgezogene Formteile aus Drahtgeflecht oder als tiefgezogene perforierte Metall- oder Kunststoffteile ausgebildet sein.

Die Wandung bzw. Abschirmung 66 kann kalotten- bzw. halbkugelförmig ausgebildet sein, wobei sich die Formstabilität beispielsweise dadurch erreichen läßt, daß eine Folie 72 aus Teflon mit glasfaserverstärktem Kunststoff zu der entsprechend geformten Wandung bzw. Abschirmung 68 verpreßt wird.

In einer anderen Ausführungsform kann die gegen mechanische Belastungen schützende Wandung bzw. Abschirmung 68 aus einem gasdurchlässigen Sinterkörper oder aus einem gasdurchlässigen Kunststoffkörper geformt werden.

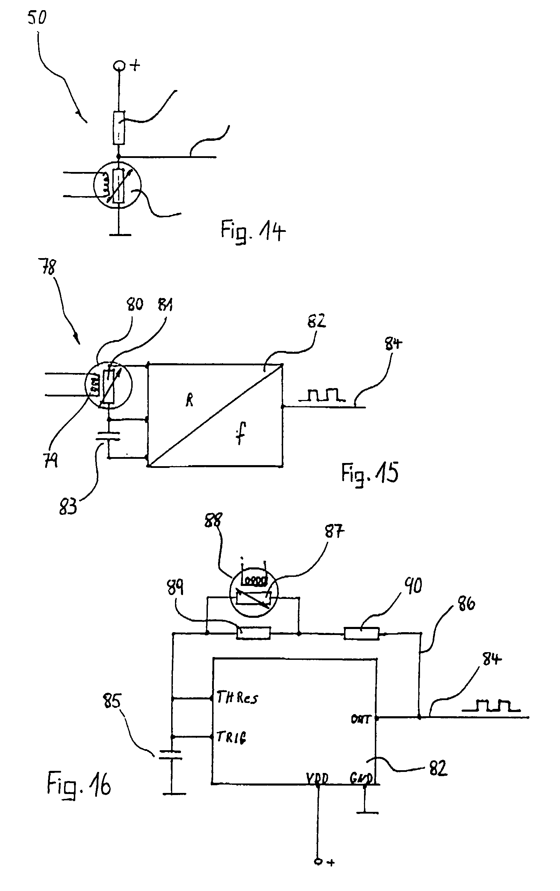

Eine Möglichkeit zur Anbringung des Gassensorelements 52 an der Leiterplatte 66 ist in den Figuren 12 und 13 dargestellt, wobei Figur 13 den Schnitt I - I in Figur 12 darstellt.

In der Leiterplatte 66 ist eine rechteckige Ausnehmung 75 ausgestaltet. Die Ausnehmung 75 befindet sich im freien Endabschnitt desjenigen Teils der Leiterplatte 66, der in die Kammer 67 vorsteht.

Im mittleren Bereich der Ausnehmung 75 der Leiterplatte 66 ist das Gassensorelement 52 angeordnet. Das Gassensorelement 52 ist mittels Anschlußdrähten 76, die aus Platin oder einem anderen geeigneten Edelmetall ausgebildet sein können, mit Anschlußstiften 77 verbunden, die auf der Leiterplatte 66 in dem die Ausnehmung 75 umgebenden Bereich vorgesehen sind. Die Halterung des Gassensorelements 52 erfolgt somit mittels der Anschlußdrähte 76 und der leiterplattenseitigen Anschlußstifte 76. Die Anschlußstifte 76 können beispielsweise aus Nickel hergestellt sein.

Die Anordnung des Gassensorelements 52 in der Ausnehmung 75 der Leiterplatte 66 ist so gewählt, daß das Gassensorelement 52 sowohl in Längs- als auch in Quer- als auch in Dickenrichtung der Leiterplatte 66 mittig in der Ausnehmung 75 sitzt.

Statt des in Figur 14 allein dargestellten Sensorteils 50, welches das Gassensorelement 52 mit dem je nach Gasbeaufschlagung veränderbaren Ohm'schen Widerstand, die Heizeinrichtung 53 und den Außenwiderstand 54 aufweist, wobei das eigentliche Gassensorsignal durch die Verbindungsleitung 55 zur nicht dargestellten Auswerteeinheit 52 geführt wird, kann auch das in Figur 15 dargstellte und im folgenden beschriebene Sensorteil 78 im Falle der erfindungsgemäßen Sensoranordnung verwendet werden. Bei dem in Figur 15 dargestellten Sensorteil 78 ist ein mittels einer Heizeinrichtung 79 beheizbares Gassensorelement 80 vorgesehen, dessen je nach Gasbeaufschlagung veränderlicher Ohm'sche Widerstand 81 mit einer Schwingschaltung 82 kombiniert ist, die als weiteren, die Frequenz bestimmenden Baustein einen Kondensator 83 aufweist. Die Schwingschaltung 82 erzeugt ein Ausgangssignal, welches durch die Leitung 84 einer nachgeschalteten Auswerteeinheit zugeführt wird.

In einer praktisch anwendbaren Ausführungsform, wie sie in Figur 16 dargestellt ist, ist die Schwingschaltung 82 als elektronischer Standardbaustein in Form beispielsweise eines Timers 555 vorgesehen. Dieser Timerbaustein ist mit einem die Frequenz bestimmenden Kondensator 85 beschaltet.

Der Timerbaustein 82 weist einen Rückkopplungszweig 86 auf. Im Rückkopplungszweig 85 ist ein je nach Gasbeaufschlagung veränderbarer Ohm'scher Widerstand 87 eines Gassensorelements 88 angeordnet, dem ein zweiter Widerstand 89 parallelgeschaltet ist. In Reihe zu der Anordnung aus den parallelgeschalteten Widerständen 87 und 89 ist im Rückkopplungszweig 86 ein dritter Widerstand 90 vorgesehen.

Durch die Parallelschaltung des zweiten Widerstands 89 zum Widerstand 87 des Gassensorelements 88 und die Reihenschaltung des dritten Widerstands zu der Anordnung aus den parallelgeschalteten Widerständen 87 und 89 wird die minimale und maximale Frequenz auch bei einer dramatischen Veränderung des Widerstands 87 des Gassensorelements 88 infolge einer plötzlichen und starken Gasbeaufschlagung begrenzt. Die Verhältnisse der drei Widerstände 87, 89 und 90 sind so gewählt, daß sowohl bei einer extremen Niederohmigkeit als auch bei einer extremen Hochohmigkeit des Widerstands 87 des Gassensorelements 88 der Gruppenwiderstand der Widerstände 87, 89, 90 stets innerhalb eines einfach zu berechnenden Bereiches bleibt, was sich unmittelbar auf die Frequenz des Ausgangssignals bezieht. Hierdurch ist sichergestellt, daß sich die Frequenz des Ausgangssignals bzw. des Gassensorsignals nie in einen Bereich verschieben könnte, der außerhalb des Aufnahmebereichs der nachgeschalteten Auswerteeinheit ist.

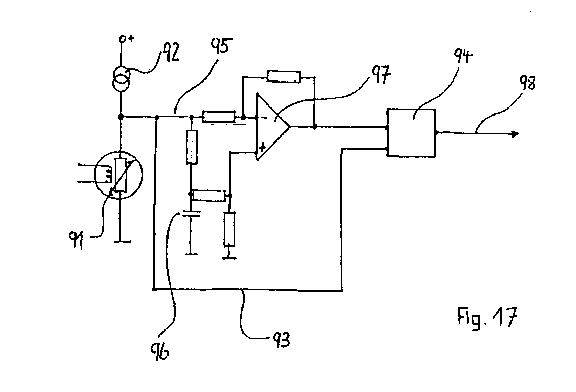

Figur 17 zeigt eine Schaltungsanordnung, mittels der die Grundbelastung der Außenluft mit Schadstoffen, die sich beispielsweise in einem ländlicher Gebiet sehr stark von der in einem innerstädtischen Gebiet unterscheidet, von impulsartig und dynamisch auftretenden Spitzenbelastungen, die beispielsweise bei einer Hinterherfahrt hinter einem dieselbetriebenen Lastkraftwagen, an einer Ampel oder an einer besonders verkehrsreichen Kreuzung auftreten können, getrennt werden kann. Bei dieser Schaltungsanordnung wird aufgrund der gemachten Erfahrungen unterstellt, daß sich die Grundbelastung nur außerordentlich langsam ändert, wohingegen sich die beispielsweise von varausfahrenden Fahrzeugen erzeugten Spitzenbelastungen außerordentlich schnell ändern.

Ein in Figur 17 dargestelltes Gassensorelement 91 wird von einer Konstantstromquelle 92 gespeist. Die sich ergebende, zwischen der Konstantstromquelle 92 und dem Gassensorelement 91 abgegriffene Gassensorspannung wird mittels einer Leitung 93 auf einen Dividierer 94 geführt.

Von der Leitung 93 zweigt eine Leitung 95 ab, mittels der das Gassensorsignal einem Integrator 96 zugeführt wird, mittels dem ein Vergleichssignal für einen Operationverstärker 97 gebildet wird. In den anderen Eingang des Operationsverstärkers 97 wird das Gassensorsignal direkt eingespeist. Das Ausgangssignal des Operationsverstärkers 97 wird ebenfalls zum Dividierer 94 geführt.

In einer Ausgangsleitung 98 des Dividierers 94 wird ein Ausgangssignal zur Verfügung gestellt, in dem die sich aufgrund von Veränderungen dar Grundbelastung langsam verändernden Signalanteile des Gassensorsignals in Abhängigkeit von der Zeitkonstante des Zeitgliedes bzw. Integrators 96 vom Gassensorsignal abgetrennt sind. Im Ausgangssignal in der Ausgangsleitung 98 des Dividierers 94 ist nur noch der etwaige dynamische Spitzenbelastungen anzeigende Signalanteil des Gassensorsignals enthalten.

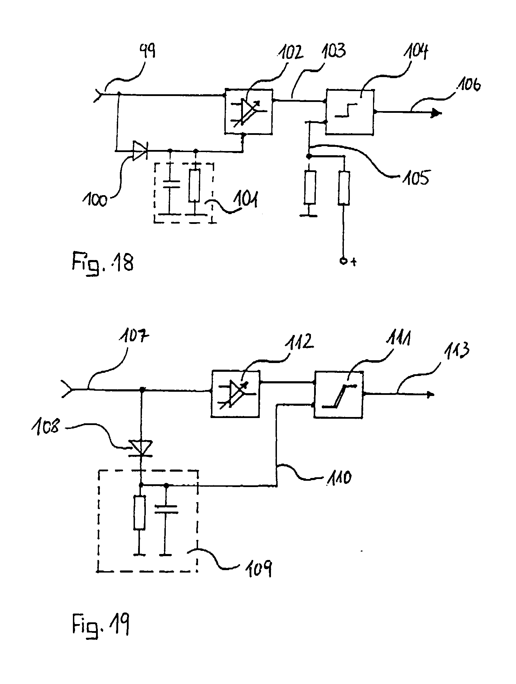

In den Figuren 18 bis 21 sind weitere Ausführungsbeispiele für Schaltungen dargestellt, mittels denen die Möglichkeit besteht, das Gassensorsignal so zu verarbeiten, daß die Ansprechempfindlichkeit der erfindungsgemäßen Sensoranordnung in Abhängigkeit von denjenigen Signalanteilen des Gassensorsignals geregelt wird, die auf die bereits mehrfach erwähnten dynamischen Spitzenbelastungen zurückgehen.

Bei dem Ausführungsbeispiel gemäß Figur 18 wird das Sensorsignal durch eine Leitung 99 einer Diode 100 zugeführt, über die das Gassensorsignal 99 ein RC-Glied 101 auflädt; hierdurch wird eine Regelspannung erzeugt, die einem regelbaren Verstärker 102 zugeführt wird.

Das Niveau des Ausgangssignals in der Ausgangsleitung 103 des regelbaren Verstärkers 102 ist daher abhängig vom Ladezustand des RC-Glieds 101, der logischerweise von der Dynamik und der Häufigkeit des Gassensorsignals abhängig ist.

Ein dem regelbaren Verstärker nachgeschalteter Schaltverstärker 104, für den die Ausgangsleitung 103 des regelbaren Verstärkers 102 als Eingangsleitung fungiert, hat einen fest eingestellten Triggerpegel, der mittels einer Eingangsleitung 105 in den Schaltverstärker 104 eingegeben wird. Der Schaltverstärker 104 erzeugt in seiner Ausgangsleitung 106 dann ein Schaltsignal, wenn der geregelte Gassensorsignalpegel das mittels des Triggerpregels vorgegebene Niveau übersteigt.

Wie im Falle der Ausführungsform gemäß Figur 18 gelangt auch im Falle der Ausführungsform gemäß Figur 19 das sogenannte dynamische Gassensorsignal, das lediglich diejenigen Signalanteile beinhaltet, die auf dynamische Belastungen des Gassensorelements aufgrund impulsartig auftretender Belastungsspitzen zurückgehen, durch eine Leitung 107 einer Diode 108 zugeführt, über die ein RC-Glied 109 aufgeladen wird. Die so mittels des RC-Glieds 109 erzeugte Spannung wird über eine Verbindungsleitung 110 einem Schaltverstärker 111 eingegeben, wodurch als Schaltverstärker 111 eine signalabhängige und insofern variable Triggerspannung anliegt.

Des weiteren wird das dynamische Gassensorsignal dem Schaltverstärker 111 über einen Verstärker 112 zugeführt, wodurch es den Schaltverstärker 111 entsprechend der Schaltschwelle, d.h. also entsprechend der vom RC-Glied erzeugten, variablen Triggerspannung, triggert. In einer Ausgangsleitung 113 des Schaltverstärkers 111 wird dann ein Schaltsignal zur Verfügung gestellt.

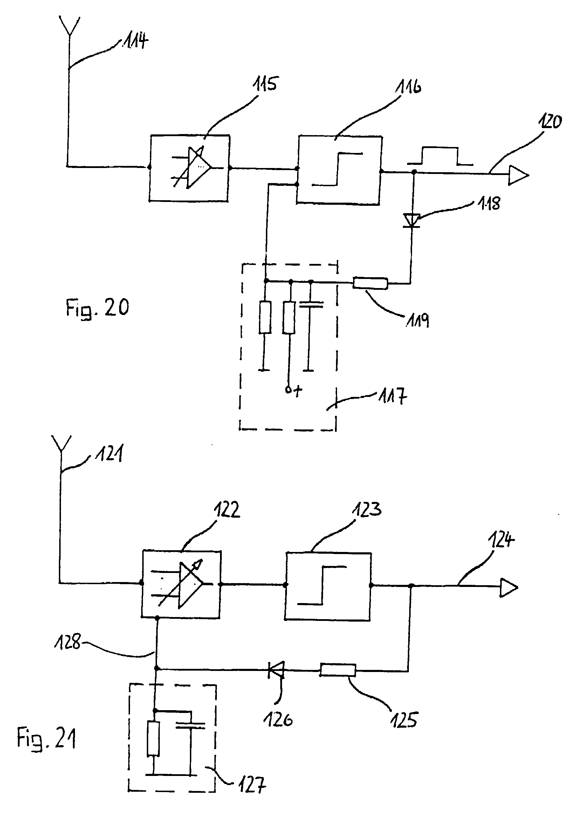

In Figur 20 ist eine weitere Ausführungsform dargestellt, bei der das dynamische Gassensorsignal, welches vorstehend erläutert und bereits mehrfach erwähnt wurde, durch eine Leitung 114 über einen Verstärker 115 einem Schaltverstärker 116 zugeführt wird.

Die Schaltspannung ergibt sich aus der Spannung eines Spannungsteilers mit einer dazugeschalteten Kondensatorschaltung 117, wobei die Spannung des in einer Ausgangsleitung 120 des Schaltverstärkers 116 vorliegenden Ausgangssignals über eine Diode 118 und einen Ladewiderstand 119 die Kondensatorschaltung 117 beeinflußt, mit der Tendenz, daß, sofern in der Ausgangsleitung 120 des Schaltverstärkers 116 häufig ein Ausgangs- bzw. Schaltsignal vorliegt, die Triggerschwelle des Schaltverstärkers 116 so verstellt wird, daß die Schaltungsanordnung "unempfindlicher" auf Änderungen des durch die Leitung 114 in sie eingegebenen dynamischen Gassensorsignals reagiert.

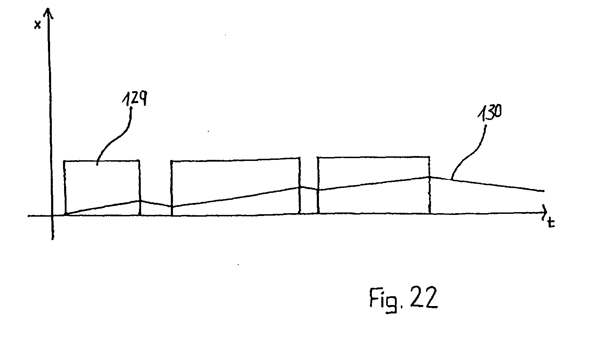

Bei der Ausführungsform gemäß Figur 21 wird - ähnlich wie im Falle der Ausführungsform gemäß Figur 20 - das dynamische Gassensorsignal durch eine Leitung 121 über einen bei dieser Ausführungsform regelbaren Verstärker 122 in einen Schaltverstärker 123 eingegeben. Das in einer Ausgangsleitung 124 vorliegende Ausgangssignal des Schaltverstärkers 123 wird über einen Ladewiderstand 125 und eine Diode 126 geführt und lädt so ein der Diode 126 nachgeschaltetes RC-Glied 127 auf, so daß dort eine Regelspannung entsteht, die über eine Verbindungsleitung 128 in den regelbaren Verstärker 122 eingegeben wird und somit die Verstärkung des regelbaren Verstärkers 122 beeinflußt.

Der dem regelbaren Verstärker 122 nachgeschaltete Schaltverstärker 123 arbeitet mit einer fest eingestellten Schaltschwelle.

In Figur 22 ist dargestellt, wie Ausgangssignale 129 des Schaltverstärkers die Ladekennlinie 130 des RC-Gliedes beeinflussen. Es wird deutlich, daß die Ladespannung eine Funktion sowohl der Häufigkeit als auch der Schaltdauer des Ausgangssignals des vorstehend erwähnten Schaltverstärkers ist.

Wie vorstehend bereits erwähnt, kann die Auswerteeinheit mit den vorstehend beschriebenen Schaltungen als zentraler programmierter Mikroprozessor ausgebildet sein, der gemäß einem entsprechend den vorstehend dargelegten Schaltkriterien ausgelegtem digital/numerischem Modell arbeitet.

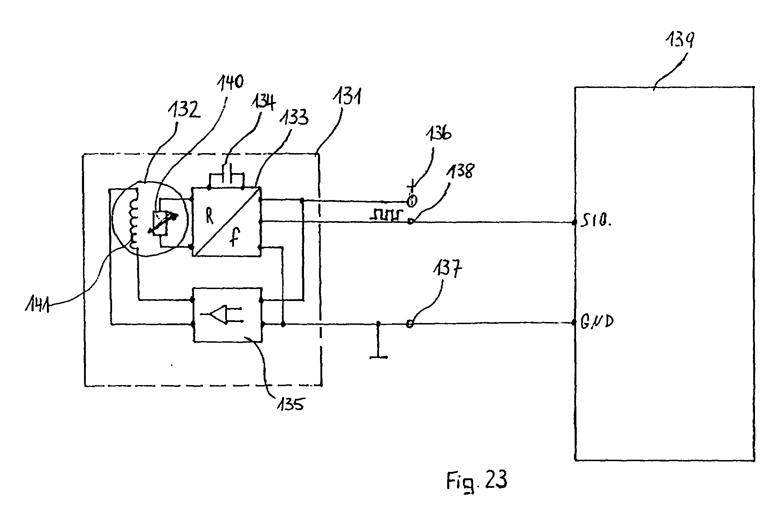

Bei der in Figur 23 dargestellten Ausführungsform der erfindungsgemäßen Sensoranordnung ist das eigentliche Sensorteil mit einer weitestgehend auf das Erforderliche reduzierten Minimalauswerteelektronik kombiniert und in einem geschützten Gehäuse angeordnet. Die eigentliche Auswertung von Daten findet in einem bei vielen Kraftfahrzeugen ohnehin vorhandenen Mikrocomputer eines zentralen Heizungs- und Klimasteuergeräts statt.

Die in Figur 23 dargestellte Ausführungsform der erfindungsgemäßen Schaltungsanordnung hat ein Sensormodul 131, das ein Gassensorelement 132, eine Schwingschaltung 133, einen die Frequenz der Schwingschaltung 133 bestimmenden Kondensator 134 und eine Heizungsregelung 135.

Das Gassensorelement 132 hat einen je nach Gasbeaufschlagung veränderbaren Ohm'schen Widerstand 140, der an die Schwingschaltung 133 angeschlossen ist, deren Frequenz durch den Kondensator 134 bestimmt wird. Mittels der Heizungaregelung 135 wird eine Heizeinrichtung 141 des Gassensorelements 132 geregelt.

Das Sensormodul 131 wird über elektrische Leitungen 136 und 137 an eine Spannungsquelle, z.B. das zentrale Heizungs- und Klimasteuergerät, angeschlossen.

Das mittels der als Rf-Glied ausgestalteten Schwingschaltung umgeformte Gassensorsignal wird über die Leitung 138 dem Mikrocomputer 139 des zentralen Heizungs- und Klimasteuergeräts eingegeben.

## Patentansprüche

1. Sensoranordnung zur Steuerung von Lüftungsanlagen in Fahrzeugen im Um- oder im Zuluftbetrieb in Abhängigkeit von der Schadstoffkonzentration in der Luft außerhalb des Fahrzeugs, mit einem Gassensorelement (52), dessen elektrischer Widerstand beim Auftreten reduzierender Gase sinkt und beim Auftreten oxidierender Gase steigt, und eine Auswerteeinheit (51), deren Ausgang an die Steuerung der Lüftungsanlage angeschlossen ist, dadurch gekennzeichnet, daß die Sensoranordnung so ausgelegt ist, daß der Anstieg eines in die Auswerteeinheit eingegebenen Gassensorsignals bei einer Erhöhung der Konzentration reduzierender Gase betragsmäßig etwa dem Abfall des in die Auswerteeinheit (51) eingegebenen Gassensorsignals bei einer entsprechenden Erhöhung der Konzentration oxidierender Gase gleichkommt, daß die Auswerteeinheit (51) aus dem in sie eingegebenen Gassensorsignal den Anstieg oder den Abfall desselben je Zeiteinheit ermittelt und daß die Auswerteeinheit (51) ein Schaltsignal zur Verstellung der Lüftungsanlage in den Umluftbetrieb erzeugt, sobald der ermittelte Anstieg oder Abfall des Gassensorsignals je Zeiteinheit betragsmäßig einen Schwellenwert übersteigt.

2. Sensoranordnung nach Anspruch 1, bei der in der Auswerteeinheit (51) aus dem Gassensorsignal für einen bestimmten Zeitraum ein Mittelwert (31) gebildet, diesem Mittelwert (31) durch einen Proportionalzu- und -abschlag bzw. die Addition und Subtraktion eines Absolutwerts ein definiertes Band (32) zugeordnet und das Schaltsignal erzeugt wird, wenn das Gassensorsignal außerhalb dieses Bandes (32) liegt.

3. Sensoranordnung nach Anspruch 1 oder 2, bei der die Auswerteeinheit (51) einen Bandpaß (57) aufweist, der ausschließlich ein Amplitudenspektrum passsieren läßt, welches einen den Schwellenwert übersteigenden Anstieg oder Abfall des Gassensorsignals je Zeiteinheit anzeigt, über den das Gassensorsignal geleitet wird, um einen derartigen Anstieg oder Abfall des Gassensorsignals je Zeiteinheit zu ermitteln und dessen Ausgangssignal zur Erzeugung der Schaltsignale für die Steuerung der Lüftungsanlage herangezogen wird.

4. Sensoranordnung nach Anspruch 1 oder 2, bei der die Auswerteeinheit (51) eine Recheneinrichtung zur Durchführung einer Fourrier-Transformation aufweist, in der das Gassensorsignal rechnerisch auf das Vorhandensein eines bestimmten, einen den Schwellenwert übersteigenden Anstieg oder Abfall des Gassensorsignals je Zeiteinheit anzeigenden Amplitudenspektrums untersucht wird, und bei der in der Auswerteeinheit (51) das Schaltsignal für die Steuerung der Lüftungsanlage erzeugt wird, wenn im Gassensorsignal ein derartiges Amplitudenspektrum ermittelt wird.

5. Sensoranordnung nach Anspruch 1 oder 2, bei der die Auswerteeinheit (51) ein elektronisches neuronales Netzwerk aufweist, in dem das Gassensorsignal nach Merkmalen auf das Vorhandensein eines den Schwellenwert übersteigenden Anstiegs oder Abfalls des Gassensorsignals je Zeiteinheit untersucht wird, und bei der in der Auswerteeinheit (51) das Schaltsignal erzeugt wird, wenn bei dieser Untersuchung ein den Schwellenwert übersteigender Anstieg oder Abfall des Gassensorsignals je Zeiteinheit ermittelt wird.

6. Sensoranordnung nach Anspruch 5, bei der das elektronische neuronale Netzwerk als drei- oder mehrschichtiges vorwärtsgekoppeltes neuronales Netzwerk ausgebildet ist.

7. Sensoranordnung nach Anspruch 5, bei der das elektronische neuronale Netzwerk als drei- oder mehrschichtiges rückwärts gekoppeltes neuronales Netzwerk ausgebildet ist.

8. Sensoranordnung nach Anspruch 1 oder 2, bei der die Auswerteeinheit (51) eine elektronische FUZZY-Logikeinheit aufweist, mittels der im Gassensorsignal ein den Schwellenwert übersteigender Anstieg oder Abfall des Gassensorsignals je Zeiteinheit erfaßbar ist, und bei der in der Auswerteeinheit (51) das Schaltsignal erzeugt wird, wenn bei dieser Untersuchung einen den Schwellenwert übersteigender Anstieg oder Abfall des Gassensorsignals je Zeiteinheit ermittelt wird.

9. Sensoranordnung nach einem der Ansprüche 1 bis 8, bei der die Auswerteeinheit (51) eine Speichereinrichtung aufweist, in der Steigungsumkehrpunkte (40, 45) des Gassensorsignals gespeichert werden, und bei der ausgangsseitig der Auswerteeinheit das Schaltsignal erlischt, wenn das Gassensorsignal nach einem Steigungsumkehrpunkt (40, 45) eine bestimmte vorgebbare Signalniveaudifferenz (42, 48) zum Steigungsumkehrpunkt (40, 45) aufweist und das Gassensorsignal innerhalb des dem gebildeten Mittelwert (31) zugeordneten Bandes (32) liegt.

10. Sensoranordnung nach einem der Ansprüche 1 bis 9, bei der die Auswerteeinheit (51) eine Regeleinrichtung zur Veränderung der Ansprechempfindlichkeit der Sensoranordnung aufweist, mittels der die Ansprechempfindlichkeit der Sensoranordnung bei steigender bzw. sinkender Häufigkeit von den Schwellenwert übersteigenden Anstiegen oder Abfällen des Gassensorsignals je Zeiteinheit reduzier- bzw. erhöhbar ist, wobei der Betrag der Veränderung der Ansprechempfindlichkeit der Sensoranordnung je Zeiteinheit begrenzt ist.

11. Sensoranordnung nach einem der Ansprüche 1 bis 10, bei der die Auswerteeinheit (51) eine Zeitmeßeinrichtung, mittels der die Betriebszeiten der Lüftungsanlage im Zuluft- und im Umluftbetrieb erfaßbar sind, eine Recheneinrichtung, mittels der der Quotient aus der Betriebszeit im Zuluftbetrieb und der Betriebszeit im Umluftbetrieb errechenbar ist, und eine Regeleinrichtung aufweist, mittels der die Ansprechempfindlichkeit der Sensoranordnung bei zunehmendem Umluftbetrieb reduziert und bei zunehmendem Zuluftbetrieb erhöht wird, wobei der Betrag der Veränderungen der Ansprechempfindlichkeit der Sensoranordnung je Zeiteinheit begrenzt ist.

12. Sensoranordnung nach Anspruch 10 oder 11, bei der die Regeleinrichtung zur Veränderung der Ansprechempfindlichkeit der Sensoranordnung einen Außentemperatursensor, der die Lufttemperatur außerhalb des Fahrzeugs erfaßt, einen Innentemperatursensor, der die Lufttemperatur innerhalb des Fahrzeugs erfaßt, und eine Vergleichseinrichtung aufweist, welche die vom Außentemperatursensor eingegebene äußere Lufttemperatur mit der vom Innentemperatursensor eingegebenen inneren Lufttemperatur vergleicht, wobei die Regeleinrichtung die Ansprechempfindlichkeit der Sensoranordnung erhöht bzw. verringert, wenn die äußere Lufttemperatur größer bzw. kleiner ist als die innere Lufttemperatur.

13. Sensoranordnung nach einem der Ansprüche 1 bis 12, bei der die Auswerteeinheit (51) mit der Steuerung der Lüftungsanlage als zentraler programmierter Mikroprozessor ausgebildet ist.

14. Sensoranordnung nach einem der Ansprüche 1 bis 12, bei der die Auswerteeinheit (51) mit der Steuerung der Lüftungsanlage analogtechnisch ausgebildet ist.

15. Sensoranordnung nach einem der Ansprüche 1 bis 14, bei der das Gassensorelement (52) als Mischoxidsensorelement ausgebildet ist, dessen gasempfindliche Schicht Zinndioxid (SnO2), Wolframtrioxid (WO3), Eisenoxid (Fe2O3), Aluminiumoxid (Al2O3) und als Reaktionabeschleuniger Platin (Pt) und Palladium (Pd) enthält.

16. Sensoranordnung nach Anspruch 15, bei der die gasempfindliche Schicht des Mischoxidsensorelements (52) 29 bis 49 % Zinndioxid (SnO2), 7 bis 13 % Eisenoxid (Fe2O3), 28 bis 48 % Wolframtrioxid (WO3), 7 bis 13 % Aluminiumoxid (Al2O3), 1 bis 3 % Palladium (Pd) und 0,5 bis 1,5 % Platin (Pt) enthält.

17. Sensoranordnung nach einem der Ansprüche 1 bis 16, bei der das Gassensorelement (52) elektrisch mit Edelmetall-, vorzugsweise Platindrähten, kontaktiert ist und in eine Ausnehmung (75) einer elektrischen Leiterplatte (66) zur Auswerteeinheit (51) mit etwa gleicher Mittelebene wie die Leiterplatte (66) eingefügt ist, und Gassensorelementanschlüsse direkt auf die Leiterplatte (66) aufgeschweißt sind, so daß das Gassensorelement (52) frei aufgehängt ist.

18. Sensoranordnung nach einem der Ansprüche 1 bis 17, bei der das Gassensorelement (52) in einer Kammer (67) angeordnet ist, die gegenüber der Auswerteeinheit (51) gasdicht abgeschlossen ist, ein kleines Volumen und eine formstabile und gasdurchlässige Wandung (68) aufweist.

19. Sensoranordnung nach Anspruch 18, bei der die Wandung (68) der Kammer (67) zumindest teilweise aus einem perforierten Werkstoff besteht, der zumindest eine Lage eines gasdurchlässigen Materials formstabil trägt und mechanisch stützt.

20. Sensoranordnung nach Anspruch 18 oder 19, bei der die zumindest eine Lage (72) des gasdurchlässigen Materials zwischen zwei Lagen (73, 74) aus Metallgewebe angeordnet ist.

21. Sensoranordnung nach Anspruch 18 oder 19, bei der die zumindest eine Lage (72) des gasdurchlässigen Materials zwischen zwei formstabilen und perforierten Lagen aus Thermoplasten eingebettet ist.

22. Sensoranordnung nach einem der Ansprüche 18 bis 21, bei der die zumindest eine Lage (72) des gasdurchlässigen Materials aus Kunststoffolie ausgebildet ist.

23. Sensoranordnung nach Anspruch 22, bei der die Kunststoffolie aus Teflon od.dgl. ausgebildet ist.

24. Sensoranordnung nach Anspruch 23, bei der die formstabile und gasdurchlässige Wandung (68) der das Gassensorelement (52) aufnehmenden Kammer (67) aus gesintertem Kunststoff, Glas, Metall od.dgl. ausgebildet ist.

25. Sensoranordnung nach einem der Ansprüche 1 bis 24, bei der das Gassensorelement (132) und eine Teileinheit (133, 134, 135) der Auswerteeinheit in einem Sensormodul (131) zusammengefaßt sind, die Teileinheit (133, 134, 135) der Auswerteeinheit eine Schwingschaltung (133), einen Kondensator (134) zur Bestimmung der Frequenz der Schwingschaltung (133) und eine Heizungsregelung (135) zur Regelung der Temperatur des Gassensorelements (132) aufweist und die Schwingschaltung (133) und die Heizungsregelung (135) an ein zentrales Heizungs- und Klimasteuergerät (139) des Fahrzeugs angeschlossen sind, wobei in dem Mikrocomputer des zentralen Heizungs- und Klimasteuergeräts (139) die Weiterverarbeitung eines Ausgangssignals des Sensormoduls (131) voll digital erfolgt.

26. Sensoranordnung nach einem der Ansprüche 1 bis 25, bei der das aus Metalloxid ausgebildete Gassensorelement mittels einer Heizeinrichtung (53) auf einer konstanten Temperatur gehalten wird.

27. Sensoranordnung nach einem der Ansprüche 1 bis 26, bei der der Ohm'sche Widerstand (140) des Gassensorelements (132) Bestandteil einer Schwingschaltung (133) ist und von einer mittelfrequenten Wechselspannung durchflossen wird.

28. Sensoranordnung nach Anspruch 27, bei der ein Kondensator (134) vorgesehen ist, der die Frequenz der Schwingschaltung (133) erzeugt.

29. Sensoranordnung nach einem der Ansprüche 26 bis 28, bei der die Schwingschaltung (82) einen Timerbaustein (82) aufweist, der mit einem frequenzbestimmenden Kondensator (85) beschaltet ist und einen Rückkopplungszweig (86) aufweist, in dem der Widerstand (87) des Gassensorelements (88) und ein zweiter Widerstand (90) angeordnet sind, und der zum Widerstand (87) des Gassensorelements (88) einen Parallelzweig aufweist, in dem ein dritter Widerstand (89) angeordnet ist.

## Claims

1. A sensor arrangement for controlling ventilation systems in vehicles in air-circulating or in air-intake operating mode in dependence on the pollutant concentration in the air outside the vehicle, having a gas sensor element (52), the electrical resistance of which drops in the presence of reducing gases and rises in the presence of oxidising gases, and having an evaluating unit (51), the output of which is connected to the control means of the ventilation system, **characterised in that** the sensor arrangement is designed so that the rate of rise of a gas sensor signal entered in the evaluating unit on increase in the concentration of reducing gases equals approximately the fall in the gas sensor signal entered in the evaluating unit (51) on corresponding increase in the concentration of oxidising gases, that the evaluating unit (51) determines from the gas sensor signal entered into it the rise or fall of the gas sensor signal per unit of time and that the evaluating unit (51) generates a switching signal for adjusting the ventilation system in air-circulating operating mode as soon as the determined rate of the rise or fall in the gas sensor signal per unit of time exceeds a threshold value.

2. A sensor arrangement according to claim 1, in which a mean value (31) is formed in the evaluating unit (51) from the gas sensor signal for a specific period, a defined band (32) is allocated to this mean value (31) by a proportional increase and decrease and respectively the addition and subtraction of an absolute value, and the switching signal is generated when the gas sensor signal lies outside this band (32).

3. A sensor arrangement according to claim 1 or 2, in which the evaluating unit (51) comprises a bandpass filter (57) which allows the passage exclusively of an amplitude spectrum that indicates a rise or fall of the gas sensor signal per unit of time exceeding the threshold value, *via* which bandpass filter the gas sensor signal is passed in order to determine such a rise or fall in the gas sensor signal per unit of time and the output signal of which is used to generate the switching signal for the control means of the ventilation system.

4. A sensor arrangement according to claim 1 or 2, in which the evaluating unit (51) comprises a computing device for performing a Fourier transformation, in which the gas sensor signal is investigated computationally for the presence of a specific amplitude spectrum indicating a rise or fall of the gas sensor signal per unit of time exceeding the threshold value, and in which the switching signal for the control means of the ventilation system is generated in the evaluating unit (51) when such an amplitude spectrum is detected in the gas sensor signal.

5. A sensor arrangement according to claim 1 or 2, in which the evaluating unit (51) comprises an electronic neural network in which the gas sensor signal is investigated for features indicating the presence of a rise or fall of the gas sensor signal per unit of time exceeding the threshold value, and in which the switching signal is generated in the evaluating unit (51) when a rise or fall of the gas sensor signal per unit of time exceeding the threshold value is detected during this examination.

6. A sensor arrangement according to claim 5, in which the electronic neural network is in the form of a three-layer or multi-layer forward-coupled neural network.

7. A sensor arrangement according to claim 5, in which the electronic neural network is in the form of a three-layer or multi-layer backward coupled neural network.

8. A sensor arrangement according to claim 1 or 2, in which the evaluating unit (51) comprises an electronic fuzzy logic unit, by means of which the rise or fall of the gas sensor signal per unit of time exceeding the threshold value is detectable in the gas sensor signal, and in which the switching signal is generated in the evaluating unit (51) whenever a rise or fall of the gas sensor signal per unit of time exceeding the threshold value is detected during this investigation.

9. A sensor arrangement according to any one of claims 1 to 8, in which the evaluating unit (51) comprises a memory device in which the gradient reversal points (40, 45) of the gas sensor signal are stored, and in which on the output side of the evaluating unit the switching signal disappears when the gas sensor signal, after a gradient reversal point (40, 45), has a specific pre-determinable signal level difference (42, 48) from the gradient reversal point (40, 45) and the gas sensor signal lies within the band (32) associated with the mean value (31) that has been formed.

10. A sensor arrangement according to any one of claims 1 to 9, in which the evaluating unit (51) comprises a controlling device for changing the responsiveness of the sensor arrangement, by means of which the responsiveness of the sensor arrangement can be reduced or increased with an increasing or reducing frequency respectively of rises or falls of the gas sensor signal per unit of time exceeding the threshold value, wherein the rate of change of the responsiveness of the sensor arrangement per unit of time is limited.

11. A sensor arrangement according to any one of claims 1 to 10, in which the evaluating unit (51) comprises a timing device, by means of which the operating times of the ventilation system in air-intake and air-circulating operating mode can be recorded, a computing device, by means of which the quotient from the operating time in air-circulating operating mode and the operating time in air-circulating operating mode can be calculated, and a controlling device device, by means of which the responsiveness of the sensor arrangement is reduced as air-circulating operating mode increases and is increased as air-intake operating mode increases, wherein the rate of change in the responsiveness of the sensor arrangement per unit of time is limited.

12. A sensor arrangement according to claim 10 or 11, in which the controlling device for changing the responsiveness of the sensor arrangement comprises an external temperature sensor, which detects the air temperature outside the vehicle, an internal temperature sensor, which detects the air temperature inside the vehicle, and a comparator, which compares the external air temperature input from the external temperature sensor with the internal air temperature input from the internal temperature sensor, wherein the controlling device increases or reduces the responsiveness of the sensor arrangement when the external air temperature is respectively greater or less than the internal air temperature.

13. A sensor arrangement according to any one of claims 1 to 12, in which the evaluating unit (51) together with the control means of the ventilation system is in the form of a central programmed microprocessor.

14. A sensor arrangement according to any one of claims 1 to 12, in which the evaluating unit (51) together with the control means of the ventilation system is of analog technical construction.

15. A sensor arrangement according to any one of claims 1 to 14, in which the gas sensor element (52) is in the form of a mixed oxide sensor element, the gas-sensitive layer of which contains stannic dioxide (SnO₂), tungsten trioxide (WO₃), iron oxide (Fe₂O₃), aluminium oxide (Al₂O₃) and as reaction accelerator platinum (Pt) and palladium (Pd).

16. A sensor arrangement according to claim 15, in which the gas-sensitive layer of the mixed oxide sensor element (52) contains 29 to 49 % of stannic dioxide (SnO₂), 7 to 13 % of iron oxide (Fe₂O₃), 28 to 48% of tungsten trioxide (WO₃), 7 to 13 % of aluminium oxide (Al₂O₃), 1 to 3 % of palladium (Pd) and 0.5 to 1.5 % of platinum (Pt).

17. A sensor arrangement according to any one of claims 1 to 16, in which the gas sensor element (52) is electrically contacted by noble metal wires, preferably platinum wires, and is inserted in a recess (75) of an electrical printed-circuit board (66) of the evaluating unit (51) with approximately the same central plane as the printed circuit board (66), and gas sensor element connections are welded directly to the printed-circuit board (66) so that the gas sensor element (52) is freely suspended.

18. A sensor arrangement according to any one of claims 1 to 17, in which the gas sensor element (52) is arranged in a chamber (67) that is sealed so that it is gas-tight with respect to the evaluating unit (51), has a small volume and a dimensionally stable and gas-permeable wall (68).

19. A sensor arrangement according to claim 18, in which the wall (68) of the chamber (67) consists at least partly of a perforated material which in a dimensionally stable manner carries and mechanically supports at least one layer of a gas-permeable material.

20. A sensor arrangement according to claim 18 or 19, in which the at least one layer (72) of the gas-permeable material is arranged between two layers (73, 74) of metallic woven fabric.

21. A sensor arrangement according to claim 18 or 19, in which the at least one layer (72) of the gas-permeable material is embedded between two dimensionally stable and perforated layers of thermoplasts.

22. A sensor arrangement according to any one of claims 18 to 21, in which the at least one layer (72) of gas-permeable material is in the form of a plastics material film.

23. A sensor arrangement according to claim 22, in which the plastics material film is formed from Teflon or similar material.

24. A sensor arrangement according to claim 23, in which the dimensionally stable and gas-permeable wall (68) of the chamber (67) receiving the gas sensor element (52) is constructed from sintered plastics material, glass, metal or similar materials.

25. A sensor arrangement according to any one of claims 1 to 24, in which the gas sensor element (132), and a sub-unit (133, 134, 135) of the evaluating unit are combined in a sensor module (131), the sub-unit (133, 134, 135) of the evaluating unit comprises an oscillating circuit (133), a capacitor (134) for determining the frequency of the oscillating circuit (133) and a heating control (135) for controlling the temperature of the gas sensor element (132), and the oscillating circuit (133) and the heating control (135) are connected to a central heating and climate control apparatus (139) of the vehicle, wherein full digital further processing of an output signal of the sensor module (131) is effected in the microcomputer of the central heating and climate control apparatus (139).

26. A sensor arrangement according to any one of claims 1 to 25, in which the gas sensor element formed from metal oxide is kept at a constant temperature by means of a heating device (53).

27. A sensor arrangement according to any one of claims 1 to 26, in which the ohmic resistance (140) of the gas sensor element (132) is a component of an oscillating circuit (133) and has a medium-frequency alternating voltage flowing through it.

28. A sensor arrangement according to claim 27, in which a capacitor (134) is provided, which generates the frequency of the oscillating circuit (133).

29. A sensor arrangement according to any one of claims 26 to 28, in which the oscillating circuit (82) comprises a timer element (82), which is wired to a frequency-determining capacitor (85) and comprises a feedback path (86) in which the resistor (87) of the gas sensor element (88) and a second resistor (90) are arranged, and which has a parallel branch, in which a third resistor (89) is arranged, to the resistor (87) of the gas sensor element (88).

## Revendications

1. Système de capteurs pour la commande d'installations de ventilation dans des véhicules, en mode recirculation d'air ou en mode admission d'air frais, en fonction de la concentration de polluants dans l'air à l'extérieur du véhicule, comprenant un élément capteur de gaz (52), dont la résistance électrique diminue en présence de gaz réducteurs et augmente en présence de gaz oxydants, et une unité de traitement (51), dont la sortie est connectée à la commande de l'installation de ventilation, caractérisé par le fait que le système de capteurs est agencé de telle sorte que la montée d'un signal de capteur de gaz transmis à l'unité de traitement lors d'une élévation de la concentration de gaz réducteurs corresponde sensiblement en valeur à la baisse du signal de capteur de gaz transmis à l'unité de traitement (51) lors d'une élévation correspondante de la concentration de gaz oxydants, que l'unité de traitement (51) détermine à partir du signal de capteur de gaz qui lui est transmis l'élévation ou la baisse de celui-ci par unité de temps et que l'unité de traitement (51) produit un signal de commutation pour amener l'installation de ventilation en mode recirculation de l'air dès que l'élévation ou la baisse déterminée du signal de capteur de gaz par unité de temps dépasse en valeur une valeur seuil.

2. Système de capteurs selon la revendication 1, dans lequel, dans l'unité de traitement (51), une valeur moyenne (31) pour une période déterminée est formée à partir du signal de capteur de gaz, une bande (32) définie est associée à ladite valeur moyenne par une majoration et une minoration proportionnelle ou par addition et soustraction d'une valeur absolue, et le signal de commutation est produit lorsque le signal de capteur de gaz se situe en dehors de cette bande (32).

3. Système de capteurs selon la revendication 1 ou 2, dans lequel l'unité de traitement (51) présente un filtre passe-bande (57) qui laisse passer exclusivement un spectre d'amplitude indiquant une élévation ou une baisse du signal de capteur de gaz par unité de temps supérieure à la valeur seuil, par lequel le signal de capteur de gaz transite aux fins de déterminer une telle élévation ou baisse de signal du capteur de gaz par unité de temps et dont le signal de sortie est utilisé pour produire les signaux de commutation pour la commande de l'installation de ventilation.

4. Système de capteurs selon la revendication 1 ou 2, dans lequel l'unité de traitement (51) comporte un dispositif de calcul pour réaliser une transformation de Fourier, dans lequel le signal de capteur de gaz est examiné par calcul sur la présence d'un spectre d'amplitude donné, indiquant une élévation ou une baisse du signal de capteur de gaz par unité de temps supérieure à la valeur seuil et dans lequel, dans l'unité de traitement (51), le signal de commutation pour la commande de l'installation de ventilation est produit lorsqu'un tel spectre d'amplitude est déterminé dans le signal de capteur de gaz.

5. Système de capteurs selon la revendication 1 ou 2, dans lequel l'unité de traitement (51) comporte un réseau électronique neuronal dans lequel le signal de capteur de gaz est examiné au niveau de ses caractéristiques sur la présence d'une élévation ou d'une baisse du signal de capteur de gaz par unité de temps supérieure à la valeur seuil et dans lequel, dans l'unité de traitement (51), le signal de commutation est produit lorsqu'au cours de cet examen une élévation ou une baisse du signal de capteur de gaz par unité de temps supérieure à la valeur seuil est déterminée.

6. Système de capteurs selon la revendication 5, dans lequel le réseau électronique neuronal est agencé en réseau neuronal dynamique à trois couches ou multicouches.

7. Système de capteurs selon la revendication 5, dans lequel le réseau électronique neuronal est agencé en réseau neuronal bouclé à trois couches ou multicouches.

8. Système de capteurs selon la revendication 1 ou 2, dans lequel l'unité de traitement (51) comporte une unité électronique de logique floue à l'aide de laquelle une élévation ou une baisse du signal de capteur de gaz par unité de temps supérieure à la valeur seuil peut être détectée dans le signal de capteur de gaz et dans lequel, dans l'unité de traitement (51), le signal de commutation est produit lorsqu'au cours de cet examen une élévation ou une baisse du signal de capteur de gaz par unité de temps supérieure à la valeur seuil est déterminée.

9. Système de capteurs selon une des revendications 1 à 8, dans lequel l'unité de traitement (51) comporte un dispositif de mémoire dans lequel des points d'inversion de pente (40, 45) du signal de capteur de gaz sont mémorisés et dans lequel, côté sortie de l'unité de traitement, le signal de commutation disparaît lorsque le signal de capteur de gaz, après un point d'inversion de pente (40, 45), présente une différence de niveau de signal (42, 48) donnée prédéterminable par rapport au point d'inversion de pente (40, 45) et le signal de capteur de gaz se situe dans la bande (32) associée à la valeur moyenne (31) formée.

10. Système de capteurs selon une des revendications 1 à 9, dans lequel l'unité de traitement (51) comporte un dispositif de réglage pour modifier la sensibilité de réaction du système de capteurs à l'aide duquel la sensibilité de réaction du système de capteurs peut être abaissée ou augmentée, lorsque la fréquence des élévations ou des baisses du signal de capteur de gaz par unité de temps supérieures à la valeur seuil augmente voire diminue, la valeur de la modification de la sensibilité de réaction du système de capteurs par unité de temps étant limitée.

11. Système de capteurs selon une des revendications 1 à 10, dans lequel l'unité de traitement (51) comporte un dispositif de mesure de temps à l'aide duquel les temps de fonctionnement du dispositif de ventilation en mode admission d'air frais et en mode recirculation d'air peuvent être mesurés, un dispositif de calcul à l'aide duquel le quotient du temps de fonctionnement en mode admission d'air frais et du temps de fonctionnement en mode recirculation d'air peut être calculé et un dispositif de réglage à l'aide duquel la sensibilité de réaction du système de capteurs est diminuée lorsque le fonctionnement en mode recirculation d'air croît et est augmentée lorsque le fonctionnement en mode admission d'air frais croît, la valeur des modifications de la sensibilité de réaction du système de capteurs par unité de temps étant limitée.

12. Système de capteurs selon la revendication 10 ou 11, dans lequel le dispositif de réglage pour modifier la sensibilité du système de capteurs comprend un capteur de température extérieure qui mesure la température de l'air à l'extérieur du véhicule, un capteur de température intérieure qui mesure la température de l'air à l'intérieur du véhicule et un dispositif comparateur qui compare la température d'air extérieure fournie par le capteur de température extérieure à la température d'air intérieure fournie par le capteur de température intérieure, le dispositif de réglage augmentant ou diminuant la sensibilité de réaction du système de capteurs lorsque la température d'air extérieure est supérieure ou inférieure à la température d'air intérieure.

13. Système de capteurs selon une des revendications 1 à 12, dans lequel l'unité de traitement (51) avec la commande de l'installation de ventilation est agencée sous forme de microprocesseur central programmé.

14. Système de capteurs selon une des revendications 1 à 12, dans lequel l'unité de traitement (51) avec la commande de l'installation de ventilation est de type analogique.

15. Système de capteurs selon une des revendications 1 à 14, dans lequel l'élément capteur de gaz (52) est agencé sous la forme d'un élément capteur à oxydes mixtes dont la couche sensible contient du dioxyde de zinc (SnO₂), du trioxyde de tungstène (WO₃), de l'oxyde de fer (Fe₂O₃), de l'oxyde d'aluminium (Al₂O₃) et, comme accélérateur de la réaction, du platine (Pt) et du palladium (Pd).

16. Système de capteurs selon la revendication 15, dans lequel la couche sensible de l'élément capteur de gaz (52) contient de 29 à 49% de dioxyde de zinc (SnO₂), de 7 à 13% d'oxyde de fer (Fe₂O₃), de 28 à 48% de trioxyde de tungstène (WO₃), de 7 à 13% d'oxyde d'aluminium (Al₂O₃), de 1 à 3% de palladium (Pd) et de 0,5 à 1,5% de platine (Pt).

17. Système de capteurs selon une des revendications 1 à 16, dans lequel l'élément capteur de gaz (52) est connecté électriquement à des fils en métal précieux, de préférence à des fils de platine, et est inséré dans un évidement (75) d'une carte à circuit imprimé (66) de l'unité de traitement (51) sensiblement avec le même plan médian que la carte à circuit imprimé (66) et les bornes de l'élément capteur de gaz sont soudées directement sur la cane à circuit imprimé (66), de telle sorte que ledit élément capteur de gaz (52) est suspendu.

18. Système de capteurs selon une des revendications 1 à 17, dans lequel l'élément capteur de gaz (52) est disposé dans une chambre (67) qui est fermée de manière étanche au gaz par rapport à l'unité de traitement (51), présente un faible volume et comporte une paroi (68) à forme stable, perméable au gaz.

19. Système de capteurs selon la revendication 18, dans lequel la paroi (68) de la chambre (67) est réalisée au moins partiellement en un matériau perforé qui porte avec stabilité de forme au moins une couche d'un matériau perméable au gaz et supporte mécaniquement celui-ci.

20. Système de capteurs selon la revendication 18 ou 19, dans lequel la couche (72) au nombre d'au moins une de matériau perméable au gaz est disposée entre deux couches (73, 74) de toile métallique.

21. Système de capteurs selon la revendication 18 ou 19, dans lequel la couche (72) au nombre d'au moins une de matériau perméable au gaz est noyée entre deux couches à forme stable, perforées, de matière thermoplastique.

22. Système de capteurs selon une des revendications 18 à 21, dans lequel la couche (72) au nombre d'au moins une de matériau perméable au gaz est formée d'un film de matière plastique.

23. Système de capteurs selon la revendication 22, dans lequel le film de matière plastique est en téflon ou une matière similaire.

24. Système de capteurs selon la revendication 23, dans lequel la paroi (68) à forme stable perméable au gaz de la chambre (67) contenant l'élément capteur de gaz (52) est réalisée en matière plastique frittée, en verre fritté, en métal fritté ou similaire.

25. Système de capteurs selon une des revendications 1 à 24, dans lequel l'élément capteur de gaz (132) et une unité élémentaire (133, 134, 135) de l'unité de traitement sont regroupés en un module capteur (131), l'unité élémentaire (133, 134, 135) de l'unité de traitement comporte un circuit oscillant (133), un condensateur (134) pour déterminer la fréquence du circuit oscillant (133) et un dispositif de régulation de chauffage (135) pour réguler la température de l'élément capteur de gaz (132) et le circuit oscillant (133) et le dispositif de régulation de température (135) sont connectés à appareil central de commande de chauffage et de climatisation (139) du véhicule, le traitement d'un signal de sortie du module capteur (131) ayant lieu de manière entièrement numérique dans le microcalculateur de l'appareil central de commande de chauffage et de climatisation (139).

26. Système de capteurs selon une des revendications 1 à 25, dans lequel l'élément capteur de gaz (132) en oxyde métallique est maintenu à une température constante au moyen d'un dispositif de chauffage (53).

27. Système de capteurs selon une des revendications 1 à 26, dans lequel la résistance ohmique (140) de l'élément capteur de gaz (132) fait partie d'un circuit oscillant (133) et est parcourue par une tension alternative à fréquence moyenne.

28. Système de capteurs selon la revendication 27, dans lequel il est prévu un condensateur (134) qui produit la fréquence du circuit oscillant (133).

29. Système de capteurs selon une des revendications 26 à 28, dans lequel le circuit oscillant (82) comporte un composant rythmeur (82) qui est connecté à un condensateur (85) déterminant la fréquence et présente une branche de rétroaction (85) dans laquelle sont insérées la résistance (87) de l'élément capteur de gaz (88) et une deuxième résistance (90) et qui présente une branche en parallèle avec la résistance (87) de l'élément capteur de gaz (88) dans laquelle est placée une troisième résistance (89).
